# FASCICULE DE BREVET EUROPEEN

(11) **EP 3 635 399 B1**
(45) Date de publication et mention de la délivrance du brevet: **29.10.2025**
(21) Numéro de dépôt: 18735659.7
(22) Date de dépôt: 01.06.2018
(51) Int. Cl.: C12N 5/071, C12N 5/09, G01N 33/569, G01N 33/574

(54) **MÉTHODE D'ISOLEMENT ET DE DÉTECTION DE CELLULES SOUCHES CANCÉREUSES**
VERFAHREN ZUR ISOLIERUNG UND ZUM NACHWEIS VON KREBSSTAMMZELLEN
METHOD FOR ISOLATING AND DETECTING CANCER STEM CELLS

(30) Priorité: 08.06.2017 FR 1755139; 08.06.2017 FR 1755137
(43) Date de publication de la demande: 15.04.2020
(73) Titulaire: Gnaho, Sylvain, 28800 Bonneval (FR)
(72) Inventeur: CARRE, Vincent, 87370 Jabreilles Les Bordes (FR); LACROIX, Aurélie, 87350 Panazol (FR)
(74) Mandataire: Oak & Fox
(86) Numéro de dépôt international: PCT/FR2018/051280
(87) Numéro de publication internationale: WO 2018/224761

(56) Documents cités:
- WO-A1-2010/126452
- WO-A1-2017/093697
- CA-A1- 2 447 400
- US-A1- 2015 147 765
- CAROL TUCKER-BURDEN ET AL: "Lectins Identify Glycan Biomarkers on Glioblastoma-Derived Cancer Stem Cells", STEM CELLS AND DEVELOPMENT, vol. 21, no. 13, 1 September 2012 (2012-09-01), pages 2374 - 2386, XP055157421, ISSN: 1547-3287, DOI: 10.1089/scd.2011.0369
- CAROL TUCKER-BURDEN ET AL: "Supplementary Table S1. Comparison of the Specificity for Monosaccharides and Oligosaccharides of a Panel of 20 Fluorescein-Isothiocyanate-Labeled Lectins Used in Flow Cytometry", 21 March 2012 (2012-03-21), XP055430080, Retrieved from the Internet <URL:http://online.liebertpub.com/doi/suppl/10.1089/scd.2011.0369/suppl_file/Supp_Table1.pdf> [retrieved on 20171129]
- S.-C. TAO ET AL: "Lectin microarrays identify cell-specific and functionally significant cell surface glycan markers", GLYCOBIOLOGY, vol. 18, no. 10, 6 June 2008 (2008-06-06), US, pages 761 - 769, XP055337468, ISSN: 0959-6658, DOI: 10.1093/glycob/cwn063
- SHINAGAWA K ET AL: "Rapid isolation of homogeneous murine bronchoalveolar lavage fluid eosinophils by differential lectin affinity interaction and negative selection", JOURNAL OF IMMUNOLOGICAL METH, ELSEVIER SCIENCE PUBLISHERS B.V.,AMSTERDAM, NL, vol. 237, no. 1-2, 1 April 2000 (2000-04-01), pages 65 - 72, XP004192495, ISSN: 0022-1759, DOI: 10.1016/S0022-1759(00)00134-4
- STEFAN GAUNITZ ET AL: "Mucin-type proteins produced in the Trichoplusia ni and Spodoptera frugiperda insect cell lines carry novel O-glycans with phosphocholine and sulfate substitutions", GLYCOBIOLOGY, vol. 23, no. 7, 5 March 2013 (2013-03-05), US, pages 778 - 796, XP055496536, ISSN: 0959-6658, DOI: 10.1093/glycob/cwt015

## Description

La présente invention concerne une méthode d'isolement et de détection de cellules souches cancéreuses (CSCs) d'organes impliqués dans la respiration.

Le cancer du poumon est la première cause de décès par cancer en France chez l'homme et la deuxième chez la femme, la première étant le cancer du sein. Il touche 37000 personnes par an. Le taux de survie à 5 ans est très faible, d'où la notion de précocité du diagnostic qui intervient dans cette pathologie où la présente invention prend tout son sens.

Le cancer du poumon comme d'autres types de cancers a de multiples facteurs induisant un taux de mortalité élevé au sein de la population concernée avec notamment un diagnostic tardif, dont découlent à la fois un cancer plus évolué dit métastatique mais aussi un fort taux de récidive. En effet, ce taux dépend directement du stade auquel est dépisté le cancer. Néanmoins, il faut savoir que même lorsque le stade de détection est précoce, le taux de récidive reste élevé car certains paramètres ne sont pour l'heure pas pris en compte.

En effet, ce phénomène de récidive peut s'expliquer en partie par la progression tumorale ainsi que les mécanismes de résistance qui reposent sur l'existence de cellules souches cancéreuses ou cellules initiatrices de tumeur ou cellules pré-cancéreuses, non prise en compte à ce jour. L'échappement thérapeutique de la tumeur aux traitements radio- et chimio-thérapeutique dépend de la présence de ces cellules au sein de la tumeur. Par conséquent, la détection de ces cellules dans le tissu tumoral constitue un moyen de définir le niveau d'agressivité de la tumeur. La caractérisation de biomarqueurs spécifiques des cellules souches cancéreuses est donc d'un grand intérêt diagnostique et pronostique dans le traitement du cancer. Cependant, il n'existe pas actuellement de marqueurs spécifiques des cellules souches cancéreuses (CSCs) qui permettent leur discrimination avec certitude des autres cellules tumorales.

Les difficultés majeures pour isoler et caractériser les CSCs résident dans la taille restreinte de leur population (3 à 4% de la population tumorale) et l'absence de marqueurs spécifiques.

La demande de brevet WO2010126452A1 propose déjà un biomarqueur, le CD166, capable de distinguer les cellules souches de cancer du poumon qui sont initiatrices de la tumorigenèse; des antagonistes aux cellules de cancer du poumon exprimant CD166 capables de diminuer la tumorigenèse du cancer du poumon à la fois in vitro et in vivo fournissant des composés pour traiter le cancer du poumon; des cellules souches et des lignées cellulaires de cancer du poumon, des sphères et du tissu de cancer du poumon pour le criblage d'antagonistes aux cellules souches de cancer du poumon.

Il existe donc un besoin important de diagnostic précoce et de développement d'une nouvelle méthode de détection et/ou d'isolement des cellules souches cancéreuses.

Une identification précoce de la présence des cellules souches cancéreuses permettrait aux cliniciens de disposer d'un facteur prédictif de la maladie.

De plus, elle offrirait de nouvelles perspectives dans le diagnostic de dangerosité du cancer. En effet, l'information supplémentaire disponible pour le clinicien devrait permettre de limiter les risques de récidive ou d'aggravation de la maladie par une adaptation du traitement.

Les cancers d'organes impliqués dans la respiration outre les poumons sont des cancers fréquents en France : il s'agit du 4ème type de cancer chez l'homme, représentant 10 % des cancers. Par ailleurs, on observe une forte disparité entre les régions du Nord et du Sud de la France, avec un taux d'incidence supérieur de plus de 20 % dans le Nord par rapport à la moyenne nationale. Il existe, aussi, de fortes inégalités sociales de mortalité.

En 2012, l'incidence des cancers d'organes impliqués dans la respiration outre le cancer des poumons est estimée à 11 320 nouveaux cas dont 71 % des cas affectent les hommes.

Par ordre de fréquence décroissant, il s'agit :
- Des cancers du pharynx : 47 %
- Des cancers de la cavité buccale, dont le cancer de la langue : 25 %
- Des cancers du larynx : 25 %
- Des cancers des glandes salivaires : 6 %
- Des cancers des sinus de la face : < 1%

Ce sont des affections qui touchent principalement les hommes (1 femme pour 7 hommes) et surtout les fumeurs.

La présente invention concerne une méthode spécifique de détection puisqu'elle ne reconnait que les cellules souches cancéreuses d'organes impliqués dans la respiration et est donc plus efficace que les méthodes classiques. De plus, sa mise en œuvre est plus rapide par rapport aux méthodes existantes, car ces dernières ne sont pas généralisables en raison de leur nonreproductibilité et regroupent à la fois des cellules souches et non souches cancéreuses.

On entend par « *organes impliqués dans la respiration* », les organes participant activement à la respiration, c'est-à-dire participant activement à l'inspiration et l'expiration, et les organes situés sur le passage de l'air respiré.

Ainsi, au sens de la présente invention, les « *organes impliqués dans la respiration* » sont les poumons, le larynx, le pharynx, la bouche, le nez, la gorge, la langue, les sinus, la trachée et les glandes salivaires (les amygdales et la glande parotide).

Dans un premier aspect, la présente invention concerne un procédé *in vitro* d'isolement de cellules souches cancéreuses d'organes impliqués dans la respiration, dans un échantillon biologique comprenant :
(a) une étape de marquage des cellules souches cancéreuses d'organes impliqués dans la respiration avec au moins une lectine choisie parmi les lectines UEA-1 ou son homologue TJA-II, ABA, ACA, jacaline, GSL-I et GSL-II, ladite lectine étant conjuguée à la biotine ou à un fluorophore, pour obtenir un échantillon biologique dans lequel les cellules souches cancéreuses d'organes impliqués dans la respiration sont marquées par au moins une lectine, suivi d'
(b) une étape d'isolement desdites cellules souches cancéreuses d'organes impliqués dans la respiration marquées par au moins une lectine,

ladite étape d'isolement étant réalisée via un support fonctionnalisé avec de la streptavidine ou de l'avidine constitué par des billes magnétiques et en présence d'un aimant, lorsque ladite lectine est conjuguée à la biotine,
et ladite étape d'isolement étant réalisée par tri cellulaire en cytométrie en flux, lorsque ladite lectine est conjuguée à un fluorophore,
dans lequel lesdits organes impliqués dans la respiration sont choisis parmi les poumons, le larynx, le pharynx, la bouche, le nez, la gorge, la langue, les sinus, la trachée et les glandes salivaires comprenant les amygdales et la glande parotide.

Dans un second aspect, la présente invention concerne l'utilisation d'au moins une lectine choisie parmi les lectines UEA-1 ou son homologue TJA-II, ABA, ACA, jacaline, GSL-I et GSL-II dans une méthode de diagnostic *in vitro* du risque de récidive du cancer d'un organe impliqué dans la respiration et/ou de l'agressivité du cancer d'un organe impliqué dans la respiration pour définir une valeur pronostique pour l'adaptation thérapeutique d'un cancer d'un organe impliqué dans la respiration,
dans lequel ledit organe impliqué dans la respiration est choisi parmi les poumons, le larynx, le pharynx, la bouche, le nez, la gorge, la langue, les sinus, la trachée et les glandes salivaires comprenant les amygdales et la glande parotide, dans laquelle la méthode de diagnostic comprend une étape de marquage des cellules souches cancéreuses d'organes impliqués dans la respiration d'un échantillon biologique d'organes impliqués dans la respiration avec au moins une lectine choisie parmi les lectines UEA-1 ou son homologue TJA-II, ABA, ACA, jacaline, GSL-I et GSL-II, pour obtenir des cellules souches cancéreuses d'organes impliqués dans la respiration marquées par au moins une lectine dans ledit échantillon.

Dans un troisième aspect, , la présente invention concerne un kit de diagnostic *in vitro* du risque de récidive du cancer d'un organe impliqué dans la respiration et/ou de l'agressivité du cancer d'un organe impliqué dans la respiration pour définir une valeur pronostique pour l'adaptation thérapeutique d'un cancer d'un organe impliqué dans la respiration, dans lequel ledit organe impliqué dans la respiration est choisi parmi les poumons, le larynx, le pharynx, la bouche, le nez, la gorge, la langue, les sinus, la trachée et les glandes salivaires comprenant les amygdales et la glande parotide, le kit comprenant au moins deux lectines choisies parmi le mélange de GSL-I et GSL-II ou le mélange UEA-1 et GSL-I, lesdites lectines étant conjuguées à la biotine, et des billes magnétiques fonctionnalisées avec de la streptavidine, et éventuellement au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, conjuguée à un fluorophore, un radio-isotope, une enzyme ou des billes d'or, ou le kit comprenant au moins deux lectines choisies parmi le mélange de GSL-I et GSL-II ou le mélange UEA-1 et GSL-I, lesdites lectines étant conjuguées à un fluorophore, et éventuellement au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, conjuguée à la biotine, un radio-isotope, une enzyme ou des billes d'or.

La présente invention repose sur la mise en évidence par les Inventeurs de l'existence du motif fucose α 1-2 galactose, et plus particulièrement du motif fucose α 1-2 galactose β 1-4 N-acétylglucosamine, à la surface des cellules souches cancéreuses d'organes impliqués dans la respiration ainsi que la possibilité de sa reconnaissance par les lectines, permettant de détecter et d'isoler ces cellules.

Au sens de la présente invention, on entend par « moyen de marquage des cellules souches cancéreuses d'organes impliqués dans la respiration » une substance capable de se lier spécifiquement à un motif à la surface des cellules souches cancéreuses d'organes impliqués dans la respiration.

Selon un aspect général, le présent texte concerne l'utilisation *in vitro* d'au moins une lectine reconnaissant le motif fucose α 1-2 galactose particulière pour le marquage de cellules souches cancéreuses d'organes impliqués dans la respiration, pour obtenir des cellules souches cancéreuses d'organes impliqués dans la respiration marquées, dans un échantillon biologique. Les lectines utilisées dans la présente invention sont choisies parmi les lectines *Ulex Europaeus agglutinin 1* (UEA-1 ) ou son homologue la lectine *Trichosanthes japonica agglutinin II* (TJA-II), *Agaricus Bisporus agglutinin* (ABA), *Amaranthus Caudatus agglutinin* (ACA), jacaline, GSL-I et GSL-II Griffonia Simplicifolia lectin I (GSL-I) et Griffonia Simplicifolia lectin II (GSL-II).

Par « au moins une lectine reconnaissant le motif fucose α 1-2 galactose particulière » on entend au moins une lectine choisie parmi les lectines UEA-1 ou son homologue TJA-II, ABA, ACA, jacaline, GSL-I et GSL-II.

Ainsi, le présent texte concerne également l'utilisation *in vitro* d'au moins une lectine choisies parmi les lectines *Ulex Europaeus agglutinin 1* (UEA-1) ou son homologue *Trichosanthes japonica agglutinin II* (TJA-II), *Agaricus Bisporus agglutinin* (ABA), *Amaranthus Caudatus agglutinin* (ACA), jacaline, GSL-I et GSL-II Griffonia Simplicifolia lectin I (GSL-I) et Griffonia Simplicifolia lectin II (GSL-II), pour obtenir des cellules souches cancéreuses d'organes impliqués dans la respiration marquées, dans un échantillon biologique. Ces lectines sont bien connues de l'homme de métier et disponibles commercialement (notamment par la société Vector Laboratories). Des revues répertorient leur structure (Lectin Structure, Rini JM, Annu Rev Biophys Biomol Struct, 1995 ; 24 : 551-77) tandis que d'autres plus récentes décrivent tout leur historique (Insight of Lectins-A review, Singh et al., International Journal of Scientific and Engineering Research, volume 3, issue 4, avril 2012) et les avancées dans leur utilisation notamment en immunohistochimie (Lectin Histochemistry : Historical Perspectives, State of the Art, and the Future, Brooks SA, Methods Mol Biol, 2017, 1560 :93-107).

Dans un mode de réalisation particulier, le présent texte concerne l'utilisation *in vitro* de la lectine UEA-1 ou son homologue TJA-II, pour obtenir des cellules souches cancéreuses d'organes impliqués dans la respiration marquées, dans un échantillon biologique.

Au sens de la présente invention, l'échantillon biologique est un échantillon prélevé sur un patient présentant un cancer d'organes impliqués dans la respiration ou susceptible de présenter un cancer d'organes impliqués dans la respiration.

Au sens de la présente invention un « *cancer d'organes impliqués dans la respiration* » peut être un cancer du poumon, un cancer du larynx, un cancer du pharynx, un cancer de la bouche, un cancer du nez, un cancer de la gorge, un cancer de la langue, un cancer des sinus, un cancer de la trachée ou un cancer des glandes salivaires (c'est-à-dire un cancer des amygdales et/ou un cancer de la glande parotide).

Dans la présente invention, les termes « cancer du poumon » et « cancer pulmonaire » peuvent être indifféremment utilisés.

Cet échantillon est susceptible de contenir des cellules souches cancéreuses.

Contrairement ou en compléments des analyses usuelles en anatomo-pathologie, l'utilisation des lectines selon la présente invention permet in fine de caractériser à un stade précoce ledit échantillon, comme étant pré-tumoral ou tumoral.

Par le terme « pré-tumoral » on entend en amont de la tumeur avec un potentiel pouvant ou non amener un caractère tumoral à l'échantillon.

En effet, l'anatomo-pathologie étudie les lésions macroscopiques et microscopiques de tissus prélevés sur des êtres vivants malades ou décédés par biopsie, frottis ou biopsie extemporanée. Cette branche de la médecine s'attache ainsi à l'étude morphologique des anomalies macroscopiques et microscopiques des tissus biologiques et des cellules pathologiques prélevées, mais pas à la recherche de cellules souches cancéreuses et donc pas aux propriétés d'auto-réplication des cellules.

L'anatomo-pathologie ne permet pas à partir d'études morphologiques, d'établir une caractérisation précoce de l'échantillon car les anomalies observées surviennent à un stade où le caractère d'auto-réplication des cellules cancéreuses est déjà exprimé.

Au contraire, la présente invention s'attachant directement à la détection de la présence de cellules souches cancéreuses, celle-ci permet de caractériser l'échantillon à un stade plus précoce que l'anatomo-pathologie, soit avant même que les cellules souches cancéreuses aient pu exprimer leur caractère d'auto-réplication entrainant les anomalies morphologiques au niveau des tissus.

La méthode selon la présente invention peut être mise en œuvre en aval d'une analyse d'anatomo-pathologie. Dans ce cas l'échantillon est caractérisé comme tumoral, susceptible d'être tumoral ou non suspecté d'être tumoral suite à l'étude d'anatomo-pathologie. La méthode selon la présente invention s'intéressant spécifiquement aux cellules souches cancéreuses, elle permet dans ce cas de confirmer le diagnostic obtenu en anatomo-pathologie, ou d'infirmer ce diagnostic.

En effet, dans le cas où un échantillon n'est pas suspecté d'être tumoral en anatomo-pathologie, la présente invention peut permettre d'infirmer ce diagnostic en révélant le caractère tumoral ou pré-tumoral dudit échantillon du fait qu'elle est basé sur des paramètres différent de l'anatomo-pathologie, en l'occurrence la présence et éventuellement la quantification de cellules souches cancéreuses.

Selon un mode de réalisation, le présent texte concerne l'utilisation *in vitro* d'un mélange d'au moins deux lectines choisies parmi UEA-1 ou son homologue TJA-II, ABA, ACA, jacaline, GSL-I et GSL-II.

Ainsi, dans un mode de réalisation, le présent texte concerne l'utilisation *in vitro* d'un mélange de deux lectines choisi parmi les mélanges suivants : (UEA-1, ABA), (UEA-1, ACA), (UEA-1, Jacaline), (UEA-1, GSL-I), (UEA-1, GSL-II), (ABA, ACA), (ABA, Jacaline), (ABA, GSL-I), (ABA, GSL-II), (ACA, Jacaline), (ACA, GSL-I), (ACA, GSL-II), (Jacaline, GSL-I), (Jacaline, GSL-II), (GSL-I, GSL-II), (TJA-II, ABA), (TJA-II, ACA), (TJA-II, Jacaline), (TJA-II, GSL-I), (TJA-II, GSL-II).

Dans un mode de réalisation particulier, le présent texte concerne l'utilisation *in vitro* d'un mélange des deux lectines GSL-I et GSL-II.

Dans un mode de réalisation particulier, le présent texte concerne l'utilisation *in vitro* d'un mélange des deux lectines GSL-I et UEA-1 ou son homologue TJA-II.

Selon un mode de réalisation, le présent texte concerne l'utilisation *in vitro* d'un mélange d'au moins trois lectines choisies parmi UEA-1 ou son homologue TJA-II, ABA, ACA, jacaline, GSL-I et GSL-II, en particulier le mélange de UEA-1 ou son homologue TJA-II, jacaline et ABA, ou le mélange de UEA-1 ou son homologue TJA-II, jacaline et ACA.

Ainsi, dans un mode de réalisation particulier, le présent texte concerne l'utilisation *in vitro* d'un mélange de trois lectines choisies parmi les mélanges suivants :
(UEA-1, ABA, ACA), (UEA-1, ABA, Jacaline), (UEA-1, ABA, GSL-I), (UEA-1, ABA, GSL-II), (UEA-1, ACA, Jacaline), (UEA-1, ACA, GSL-I), (UEA-1, ACA, GSL-II), (UEA-1, Jacaline, GSL-I), (UEA-1, Jacaline, GSL-II), (UEA-1, GSL-I, GSL-II), (ABA, ACA, Jacaline), (ABA, ACA, GSL-I), (ABA, ACA, GSL-II), (ABA, Jacaline, GSL-I), (ABA, Jacaline, GSL-II), (ABA, GSL-I, GSL-II), (ACA, Jacaline, GSL-I), (ACA, Jacaline, GSL-II), (ACA, GSL-I, GSL-II), (Jacaline, GSL-I, GSL-II), (TJA-II, ABA, ACA), (TJA-II, ABA, Jacaline), (TJA-II, ABA, GSL-I), (TJA-II, ABA, GSL-II), (TJA-II, ACA, Jacaline), (TJA-II, ACA, GSL-I), (TJA-II, ACA, GSL-II), (TJA-II, Jacaline, GSL-I), (TJA-II, Jacaline, GSL-II), (TJA-II, GSL-I, GSL-II).

Dans un mode de réalisation, le présent texte concerne l'utilisation *in vitro* du mélange des trois lectines (UEA-1, jacaline, ABA), ou du mélange de (UEA-1, jacaline, ACA).

L'utilisation de deux ou trois lectines permet dans certains cas une meilleure spécificité du marquage des cellules souches cancéreuses.

La combinaison des deux GSL ou de UEA-1 et GSL-I ou de TJA-II et GSL-I sont des modes de réalisation avantageux dans la détection et l'isolement des CSCs.

Dans un mode de réalisation particulier, le présent texte concerne l'utilisation *in vitro* d'au moins une lectine reconnaissant le motif fucose α 1-2 galactose pour le marquage de cellules souches cancéreuses pulmonaires, choisie parmi les lectines UEA-1 ou son homologue TJA-II, ABA, ACA, jacaline, GSL-I et GSL-II, pour obtenir des cellules souches cancéreuses pulmonaires marquées, dans un échantillon biologique,
notamment au moins deux lectines choisies parmi UEA-1, ABA, ACA,jacaline, GSL-I et GSL-II, en particulier le mélange de GSL-I et GSL-II ou le mélange UEA-1 et GSL-I, notamment au moins trois lectines choisies parmi UEA-1, ABA, ACA, jacaline, GSL-I et GSL-II, en particulier le mélange de UEA-1, jacaline et ABA, ou le mélange de UEA-1, jacaline et ACA.

La lectine utilisée dans le cadre de l'invention peut être conjuguée.

Au sens de l'invention, on entend par le terme « conjuguée » que la lectine est liée de manière covalente à une autre molécule.

Selon un mode de réalisation, le présent texte concerne l'utilisation *in vitro* d'au moins une lectine reconnaissant le motif fucose α 1-2 galactose particulière pour le marquage de cellules souches cancéreuses d'organes impliqués dans la respiration, dans laquelle ledit marquage de cellules souches cancéreuses d'organes impliqués dans la respiration est réalisé avec une lectine conjuguée à un marqueur choisi parmi : un fluorophore, un radio-isotope, une enzyme, des billes d'or ou la biotine.

Ainsi, dans un mode de réalisation particulier, ladite lectine est conjuguée à un fluorophore. Au sens de l'invention, un fluorophore peut être tout fluorophore susceptible d'être utilisé pour la cytométrie en flux. De tels fluorophores sont disponibles dans le commerce. Il s'agit par exemple de l'Alexa fluor, en particulier l'Alexa fluor 350, 405, 430, 488, 500, 514, 532, 546, 555, 568, 594, 610, 633, 647, 660, 680, 700, 750 ou 790, l'isothiocyanate de fluorescéine (FITC), la Rhodamine, l'allophycocyanine (APC) et la Phycoérythrine (PE).

Avantageusement, le fluorophore est choisi parmi la rhodamine, le FITC ou l'Alexa fluor, en particulier l'Alexa fluor 488, l'Alexa fluor 594 ou l'Alexa fluor 633.

Cette caractérisation du fluorophore au sens de l'invention, s'applique à tout mode de réalisation de la présente invention faisant intervenir un fluorophore.

Dans un autre mode de réalisation particulier, ladite lectine est conjuguée à un radio-isotope.

Au sens de l'invention, un radio-isotope est choisi parmi l'iode 125, le tritium ou le technétium. Dans un autre mode de réalisation particulier, ladite lectine est conjuguée à une enzyme.

Au sens de l'invention, l'enzyme est une enzyme catalysant la formation d'un produit coloré, c'est-à-dire une enzyme utilisant un substrat chromogène, ou une enzyme catalysant la formation d'un produit luminescent, c'est-à-dire une enzyme utilisant un substrat chimioluminescent.

Au sens de l'invention, un « substrat chromogène » désigne un substrat donnant un produit coloré après conversion par une enzyme.

Au sens de l'invention un « substrat chimioluminescent » désigne un substrat donnant un produit luminescent après conversion par une enzyme.

Dans un cas particulier de l'invention, ladite enzyme catalysant la formation d'un produit coloré est choisie parmi la peroxydase de raifort (HRP), la phosphatase alcaline, la glucose oxydase ou la β-galactosidase.

Dans le cas particulier de la HRP, le substrat chromogène est choisi parmi 3,3'-Diaminobenzidine (DAB), 3,3',5,5'-Tetramethylbenzidine (TMB), ou 2,2'-azino-bis(acide 3-ethylbenzothiazoline-6-sulphonique) (ABTS).

Dans le cas particulier de la phosphatase alcaline, le substrat chromogène est le NBT (Nitrobleu de tetrazolium) et le BCIP (bromochlorylindolophosphate).

Dans un cas particulier de l'invention, ladite enzyme catalysant la formation d'un produit luminescent est la HRP et le substrat luminescent est le luminol.

Dans un autre mode de réalisation particulier, ladite lectine est conjuguée à des billes d'or.

Dans un autre mode de réalisation particulier, ladite lectine est conjuguée à la biotine.

Il a également été mis en évidence par les Inventeurs que les cellules souches cancéreuses d'organes impliqués dans la respiration pouvaient être détectées via l'utilisation d'une lectine reconnaissant le motif fucose α 1-2 galactose particulière pour le marquage desdites cellules souches cancéreuses d'organes impliqués dans la respiration.

Au sens de la présente invention, on entend par « détection » le fait d'identifier par des méthodes d'UV/visible, de luminescence, de fluorescence, de radioactivité, d'enzymologie la présence de cellules souches cancéreuses d'organes impliqués dans la respiration au sein d'un échantillon biologique.

Ainsi, le présent texte concerne également l'utilisation d'au moins une lectine reconnaissant le motif fucose α 1-2 galactose particulière, , pour le marquage des cellules souches cancéreuses d'organes impliqués dans la respiration suivi de la détection des cellules souches cancéreuses dans un échantillon biologique, via la détection de ladite lectine conjuguée.

Dans un mode de réalisation, la lectine peut être conjuguée de manière covalente à un fluorophore.

Ainsi, selon un mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée à un fluorophore et est suivi de la détection desdites cellules souches cancéreuses d'organes impliqués dans la respiration par microscopie à fluorescence ou par lecteur de fluorescence.

Dans un mode de réalisation, la lectine peut être conjuguée à un radio-isotope.

Ainsi, selon un mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée à un radio-isotope et est suivi de la détection desdites cellules souches cancéreuses d'organes impliqués dans la respiration marquées par une gamma caméra. Dans un mode de réalisation, la lectine peut être conjuguée à une enzyme utilisant un substrat chromogène ou un substrat chimioluminescent.

Ainsi, selon un mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée à la péroxydase de raifort et est suivi de la détection desdites cellules souches cancéreuses d'organes impliqués dans la respiration marquées par microscopie à luminescence ou par un lecteur de luminescence par ajout d'un substrat chimio luminescent, tel que le luminol.

Dans un autre mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée à la péroxydase de raifort et est suivi de la détection desdites cellules souches cancéreuses d'organes impliqués dans la respiration marquées par microscopie UV/visible ou par lecteur d'absorbance, via l'ajout d'un substrat chromogène choisi parmi 3,3'-Diaminobenzidine (DAB), 3,3',5,5'-Tetramethylbenzidine (TMB), ou 2,2'-azino-bis(acide 3-ethylbenzothiazoline-6-sulphonique) (ABTS).

Dans un mode de réalisation, la lectine peut être conjuguée à des billes d'or.

Ainsi, selon un mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée à des billes d'or et est suivi
de la détection desdites cellules souches cancéreuses d'organes impliqués dans la respiration marquées par microscopie électronique.

Dans un mode de réalisation, la lectine peut être conjuguée à la biotine, pour donner une lectine biotinylée.

Ainsi, selon un mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée à la biotine et est suivi

de la détection desdites cellules souches cancéreuses d'organes impliqués dans la respiration marquées par la lectine conjuguée à la biotine par l'un des modes précédemment décrit dans le quel ledit marqueur, fluorophore, radio-isotope, enzyme, billes d'or, est lui-même conjugué avec de la streptavidine ou de l'avidine.

Lorsque le marquage de cellules souches cancéreuses d'organes impliqués dans la respiration est réalisé avec une lectine conjuguée à la biotine et est suivi
de la détection desdites cellules souches cancéreuses d'organes impliqués dans la respiration marquées par la lectine conjuguée à la biotine, la détection est faite :
o par microscopie à fluorescence lors de l'utilisation d'un fluorophore conjugué à de la streptavidine ou à de l'avidine,
o par lecteur de luminescence lors de l'utilisation d'une enzyme utilisant un substrat chimioluminescent conjuguée à de la streptavidine ou à de l'avidine
∘ par gamma caméra lors de l'utilisation d'un radio-isotope conjugué à de la streptavidine ou à de l'avidine,
∘ par microscopie électronique lors de l'utilisation de billes d'or conjuguées à de la streptavidine ou à de l'avidine,
∘ par microscopie UV/visible lors de l'utilisation d'une enzyme utilisant un substrat chromogène conjuguée à de la streptavidine ou à de l'avidine.

Il a également été mis en évidence par les Inventeurs que les cellules souches cancéreuses d'organes impliqués dans la respiration pouvaient être isolées via l'utilisation d'une lectine reconnaissant le motif fucose α 1-2 galactose particulière pour le marquage desdites cellules souches cancéreuses d'organes impliqués dans la respiration.

Par « isolement des cellules souches cancéreuses d'organes impliqués dans la respiration » on entend l'extraction des cellules souches cancéreuses d'organes impliqués dans la respiration d'un échantillon biologique, débarrassées de tout autre type cellulaire.

Ainsi, le présent texte concerne également l'utilisation d'au moins une lectine reconnaissant le motif fucose α 1-2 galactose particulière, pour le marquage des cellules souches cancéreuses d'organes impliqués dans la respiration suivi de l'isolement des cellules souches cancéreuses dans un échantillon biologique, ladite lectine étant conjuguée.

Cet isolement permet un enrichissement de l'échantillon en cellules souches cancéreuses d'organes impliqués dans la respiration.

Par le terme « enrichissement », on entend que la proportion des cellules souches cancéreuses d'organes impliqués dans la respiration par rapport aux cellules totales contenues dans l'échantillon est augmentée, du fait de la déplétion de l'échantillon en cellules non souches cancéreuses.

On parle alors d'un échantillon enrichi en cellules souches cancéreuses d'organes impliqués dans la respiration.

Ainsi par l'expression « isolement des cellules souches cancéreuses d'organes impliqués dans la respiration » dans le contexte de l'invention, on entend « enrichissement de l'échantillon en cellules souches cancéreuses d'organes impliqués dans la respiration ».

Ainsi, au sens de la présente invention, on entend également par « isolement » le fait d'obtenir une population de cellules enrichie en cellules souches cancéreuses d'organes impliqués dans la respiration à partir d'un échantillon biologique. Le terme « enrichi » désigne au sens de la présente invention une population de cellules dans laquelle le rapport nombre de cellules souches cancéreuses / nombre total de cellules est d'au moins 8 tel que déterminé par le rapport cellules Epcam high + / cellules Epcam high - par cytométrie en flux.

L'enrichissement de l'échantillon en cellules souches cancéreuses permet une détection et une quantification plus fiable et plus aisée des cellules souches cancéreuses du fait que la population cellulaire recherchée est alors présente en plus grande proportion dans l'échantillon.

Ainsi, il a été mis en évidence par les Inventeurs qu'un échantillon biologique pouvait être enrichi en cellules souches cancéreuses de manière particulièrement efficace en utilisant une lectine reconnaissant le motif fucose α 1-2 galactose particulière.

Dans un mode de réalisation particulier, l'isolement des cellules souches cancéreuses marquées par au moins une lectine reconnaissant le motif fucose α 1-2 galactose particulière, peut être suivie d'une étape d'amplification cellulaire. Ainsi, après l'isolement des cellules, celles-ci peuvent être mises en culture dans un milieu permettant d'augmenter la quantité de cellules souches cancéreuses d'organes impliqués dans la respiration.

Dans un mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée et est suivi de l'isolement desdites cellules souches cancéreuses d'organes impliqués dans la respiration marquées.

Dans un mode particulier de réalisation, la lectine est conjuguée à la biotine et l'isolement des cellules souches cancéreuses d'organes impliqués dans la respiration marquées est réalisé via un support fonctionnalisé avec de la streptavidine ou de l'avidine.

Dans ce mode de réalisation, les cellules souches cancéreuses d'organes impliqués dans la respiration marquées avec une lectine conjuguée à la biotine, sont fixées sur le support fonctionnalisé avec de la streptavidine ou de l'avidine, par l'affinité biotine-streptavidine ou biotine-avidine.

Le support peut également être en verre, en polydiméthylsiloxane (PDMS), en silicone, ou en plastique tel que le polyméthylméthacrylate (PMMA), le polystyrène (PS) ou en copolymère cyclique d'oléfines (COC).

Des exemples de supports appropriés sont donnés dans la revue de Kim *et al.* (Protein immobilization techniques for microfluidics assays, Kim et al., Biomicrofluidics, 7, 041501, 2013).

Par le terme « fonctionnalisé », on entend que le support est chimiquement modifié pour être recouvert de streptavidine ou d'avidine immobilisée.

La revue Kim *et al.,* précédemment citée, donne des exemples de fonctionnalisation de support.

Dans un mode de réalisation plus particulier, ledit support est constitué de billes magnétiques.

Ainsi, selon un mode particulier de réalisation de l'invention, ledit support est constitué de billes magnétiques et l'isolement desdites cellules souches cancéreuses d'organes impliqués dans la respiration marquées est réalisé par tri magnétique en présence d'un aimant.

Dans ce mode de réalisation, les cellules souches cancéreuses d'organes impliqués dans la respiration avec une lectine conjuguée à la biotine, sont fixées sur les billes magnétiques fonctionnalisées avec de la streptavidine ou de l'avidine, par l'affinité biotine-streptavidine ou biotine-avidine.

Sous l'effet d'un aimant, les cellules souches cancéreuses d'organes impliqués dans la respiration fixées sur les billes magnétiques sont isolées au sein de l'échantillon.

Cet isolement peut être suivi de la récupération de l'échantillon enrichi en cellules souches cancéreuses, par élimination du surnageant, puis par élution des cellules souches cancéreuses liées audit support.

Ladite élution peut être réalisée en condition acide pour rompre la liaison streptavidine- biotine ou avidine-biotine.

Dans le cas particulier où ledit support est constitué par des billes magnétiques et où la streptavidine ou l'avidine est liée aux billes magnétiques par une liaison ADN, ladite élution est réalisée par traitement à la DNAse.

Selon un autre mode de réalisation, la lectine utilisée pour le marquage est une lectine conjuguée à un fluorophore et l'isolement est réalisé par tri cellulaire en cytométrie en flux.

Le tri cellulaire par cytométrie en flux permet ainsi d'obtenir une fraction de l'échantillon enrichie en cellules souches cancéreuses d'organes impliqués dans la respiration.

La cytométrie en flux est une technique bien connue de l'Homme du métier qui permet notamment de trier les cellules en différentes fractions en fonction de leur marquage fluorescent.

Le tri cellulaire par cytométrie en flux dans le cadre de l'invention permet ainsi d'obtenir :
- d'une part, une fraction de l'échantillon comportant les cellules souches cancéreuses d'organes impliqués dans la respiration marquées par une lectine conjuguée à un fluorophore, et
- d'autre part, une fraction de l'échantillon contenant les autres types cellulaires contenus dans l'échantillon de départ.

L'invention permet également le marquage, l'isolement puis la détection de cellules souches cancéreuses d'organes impliqués dans la respiration en utilisant au moins une lectine conjuguée.

Dans un autre mode de réalisation, le présent texte concerne l'utilisation *in vitro* d'au moins une lectine reconnaissant le motif fucose α 1-2 galactose particulière pour le marquage de cellules souches cancéreuses d'organes impliqués dans la respiration, dans laquelle ledit marquage de cellules souches cancéreuses d'organes impliqués dans la respiration avec une lectine conjuguée est suivi de l'isolement desdites cellules souches cancéreuses d'organes impliqués dans la respiration marquées
puis de la détection desdites cellules souches cancéreuses d'organes impliqués dans la respiration isolées, via un nouveau marquage desdites cellules souches cancéreuses d'organes impliqués dans la respiration isolées
avec une lectine conjuguée à un marqueur choisi parmi : un fluorophore, un radio-isotope, une enzyme, des billes d'or ou la biotine.

Ainsi dans un autre mode de réalisation, le présent texte concerne l'utilisation *in vitro* d'au moins une lectine reconnaissant le motif fucose α 1-2 galactose particulière, pour le marquage de cellules souches cancéreuses d'organes impliqués dans la respiration, dans laquelle ledit marquage de cellules souches cancéreuses d'organes impliqués dans la respiration est réalisé avec une lectine conjuguée à une biotine ou à un fluorophore et est suivi de
- l'isolement desdites cellules souches cancéreuses d'organes impliqués dans la respiration marquées
   via un support fonctionnalisé avec de la streptavidine ou de l'avidine dans le cas d'une lectine conjuguée à la biotine comme décrit dans la présente invention, ou
   via la cytométrie en flux dans le cas d'une lectine conjuguée avec un fluorophore, comme décrit dans la présente invention,
   pour obtenir des cellules souches cancéreuses d'organes impliqués dans la respiration marquées et isolées,
- puis d'un nouveau marquage avec une lectine conjuguée selon l'invention, desdites cellules souches cancéreuses d'organes impliqués dans la respiration marquées et isolées, suivi de la détection desdites cellules selon les modes de détection décrits dans la présente demande.

Selon un mode de réalisation de la présente invention ledit échantillon biologique à partir duquel les cellules souches cancéreuses sont isolées ou détectées est un échantillon biologique d'organes impliqués dans la respiration.

L'échantillon biologique d'organes impliqués dans la respiration peut notamment être une biopsie tumorale prélevée chez un patient atteint d'un cancer des d'organes impliqués dans la respiration ou une biopsie prélevée chez un patient suspecté d'avoir un tel cancer.

L'échantillon biologique d'organes impliqués dans la respiration peut également être une lignée cellulaire cancéreuse d'organes impliqués dans la respiration ou une tumeur induite chez un animal par injection de lignées cellulaires cancéreuses, par exemple chez la souris ou le rat. La lignée cellulaire est de préférence une lignée cellulaire cancéreuse d'organes impliqués dans la respiration. Selon ce mode de réalisation, la tumeur induite contient des cellules souches cancéreuses d'organes impliqués dans la respiration qui sont avantageusement isolées des autres cellules de la tumeur afin d'être étudiées.

Selon un mode de réalisation, le présent texte concerne l'utilisation *in vitro* d'au moins une lectine reconnaissant le motif fucose α 1-2 galactose particulière pour le marquage de cellules souches cancéreuses d'organes impliqués dans la respiration, pour obtenir des cellules souches cancéreuses d'organes impliqués dans la respiration marquées, dans un échantillon biologique, dans laquelle ledit échantillon biologique est constitué par des cellules en suspension.

Ladite au moins une lectine est choisie parmi les lectines UEA-1 ou son homologue TJA-II, ABA, ACA, jacaline, GSL-I et GSL-II.

Ainsi, le présent texte concerne l'utilisation *in vitro* d'au moins une lectine choisie parmi les lectines UEA-1 ou son homologue TJA-II, ABA, ACA, jacaline, GSL-I et GSL-II pour le marquage de cellules souches cancéreuses d'organes impliqués dans la respiration, pour obtenir des cellules souches cancéreuses d'organes impliqués dans la respiration marquées, dans un échantillon biologique, dans laquelle ledit échantillon biologique est constitué par des cellules en suspension.

Selon un autre mode de réalisation, le présent texte concerne l'utilisation *in vitro* d'au moins une lectine reconnaissant le motif fucose α 1-2 galactose particulière pour le marquage de cellules souches cancéreuses d'organes impliqués dans la respiration, pour obtenir des cellules souches cancéreuses d'organes impliqués dans la respiration marquées, dans un échantillon biologique, dans laquelle ledit échantillon biologique est constitué par un tissu cellulaire.

Ainsi, le présent texte concerne l'utilisation *in vitro* d'au moins une lectine choisie parmi les lectines UEA-1 ou son homologue TJA-II, ABA, ACA, jacaline, GSL-I et GSL-II, pour le marquage de cellules souches cancéreuses d'organes impliqués dans la respiration, pour obtenir des cellules souches cancéreuses d'organes impliqués dans la respiration marquées, dans un échantillon biologique, dans laquelle ledit échantillon biologique est constitué par un tissu cellulaire.

Selon un mode de réalisation, le présent texte concerne l'utilisation *in vitro,* telle que décrite ci-dessus, d'au moins deux lectines, lesdites au moins deux lectines étant en quantité équimolaire.

Selon un mode de réalisation, le présent texte concerne l'utilisation *in vitro* d'au moins deux lectines choisi parmi les lectines UEA-1 ou son homologue TJA-II, ABA, ACA, jacaline, GSL-I et GSL-II, lesdites au moins deux lectines étant en quantité équimolaire.

Selon un mode de réalisation, le présent texte concerne l'utilisation *in vitro* d'un mélange de deux lectines choisies parmi les lectines UEA-1 ou son homologue TJA-II, ABA, ACA, jacaline, GSL-I et GSL-II, lesdites deux lectines étant en quantité équimolaire dans ledit mélange.

Par quantité équimolaire, on entend que chacune des deux lectines est utilisée en quantité identique par rapport à l'autre. Il s'agit d'un ratio en poids de 1 :1 entre les deux lectines.

Ainsi, dans un mode de réalisation, le présent texte concerne l'utilisation *in vitro* d'un mélange de deux lectines choisi parmi les mélanges suivants : (UEA-1, ABA), (UEA-1, ACA), (UEA-1, Jacaline), (UEA-1, GSL-I), (UEA-1, GSL-II), (ABA, ACA), (ABA, Jacaline), (ABA, GSL-I), (ABA, GSL-II), (ACA, Jacaline), (ACA, GSL-I), (ACA, GSL-II), (Jacaline, GSL-I), (Jacaline, GSL-II), (GSL-I, GSL-II), (TJA-II, ABA), (TJA-II, ACA), (TJA-II, Jacaline), (TJA-II, GSL-I), (TJA-II, GSL-II), lesdites deux lectines étant en quantité équimolaire dans ledit mélange.

Selon un mode de réalisation, le présent texte concerne l'utilisation *in vitro* de deux lectines, lesdites deux lectines étant un mélange (GSL-I, GSL-II), dans lequel chacune des lectines sont en quantité équimolaire.

Selon un mode de réalisation, le présent texte concerne l'utilisation *in vitro* de deux lectines, lesdites deux lectines étant un mélange (UEA-1, GSL-I), dans lequel chacune des lectines sont en quantité équimolaire.

Selon un mode de réalisation, le présent texte concerne l'utilisation *in vitro,* telle que décrite ci-dessus, d'au moins deux lectines, lesdites au moins deux lectines étant en quantité non équimolaire.

Selon un mode de réalisation, le présent texte concerne l'utilisation *in vitro* d'au moins deux lectines choisies parmi les lectines UEA-1 ou son homologue TJA-II, ABA, ACA, jacaline, GSL-I et GSL-II, lesdites au moins deux lectines étant en quantité non équimolaire.

Selon un mode de réalisation, le présent texte concerne l'utilisation *in vitro* d'un mélange de deux lectines choisies parmi les lectines UEA-1 ou son homologue TJA-II, ABA, ACA, jacaline, GSL-I et GSL-II lesdites deux lectines étant en quantité non équimolaire dans ledit mélange.

Par quantité non équimolaire, on entend que chacune des lectines est présente en quantité différente par rapport à l'autre. Il s'agit en particulier d'un ratio en poids de 2 :1, 3 :1 ou 4 :1 entre les deux lectines.

Ainsi, dans un mode de réalisation, le présent texte concerne l'utilisation *in vitro* d'un mélange de deux lectines choisi parmi les mélanges suivants : (UEA-1, ABA), (ABA, UEA-1), (UEA-1, ACA), (ACA, UEA-1), (UEA-1, Jacaline), (Jacaline, UEA-1), (UEA-1, GSL-I), (GSL-I, UEA-1), (UEA-1, GSL-II), (GSL-II, UEA-1), (ABA, ACA), (ACA, ABA), (ABA, Jacaline), (Jacaline, ABA), (ABA, GSL-I), (GSL-I, ABA), (ABA, GSL-II), (GSL-II, ABA), (ACA, Jacaline), (Jacaline, ABA), (ACA, GSL-I), (GSL-I, ACA), (ACA, GSL-II), (GSL-II, CA), (Jacaline, GSL-I), (GSL-I, Jacaline), (Jacaline, GSL-II), (GSL-II, Jacaline), (GSL-I, GSL-II), (GSL-II, GSL-I), (TJA-II, ABA), (ABA, TJA-II), (TJA-II, ACA), (ACA, TJA-II), (TJA-II, Jacaline), (Jacaline, TJA-II), (TJA-II, GSL-I), (GSL-I, TJA-II), (TJA-II, GSL-II), (GSL-II, TJA-II), lesdites deux lectines étant en quantité non équimolaire dans ledit mélange, en particulier dans un ratio en poids de 2 :1, 3 :1 ou 4 :1, plus particulièrement 2 :1.

Selon un mode de réalisation particulier, le présent texte concerne l'utilisation *in vitro* de deux lectines, lesdites deux lectines étant un mélange (UEA-1, GSL-I), dans lequel les lectines sont en quantité non équimolaire dans un ratio en poids de 2 : 1 soit 2 UEA-1 pour 1 GSL-I.

Selon un mode de réalisation particulier, le présent texte concerne l'utilisation *in vitro* de deux lectines, lesdites deux lectines étant un mélange (UEA-1, GSL-I), dans lequel les lectines sont en quantité non équimolaire dans un ratio en poids de 3 : 1 soit 3 UEA-1 pour 1 GSL-I.

Selon un mode de réalisation particulier, le présent texte concerne l'utilisation *in vitro* de deux lectines, lesdites deux lectines étant un mélange (UEA-1, GSL-I), dans lequel les lectines sont en quantité non équimolaire dans un ratio en poids de 4 : 1 soit 4 UEA-1 pour 1 GSL-I.

Selon un mode de réalisation, le présent texte concerne l'utilisation *in vitro* d'au moins trois lectines choisies parmi les lectines UEA-1 ou son homologue TJA-II, ABA, ACA, jacaline, GSL-I et GSL-II, lesdites au moins trois lectines étant en quantité équimolaire.

Selon un mode de réalisation, le présent texte concerne l'utilisation *in vitro* d'un mélange de trois lectines choisies parmi les lectines UEA-1 ou son homologue TJA-II, ABA, ACA, jacaline, GSL-I et GSL-II, lesdites trois lectines étant chacune en quantité équimolaire dans ledit mélange.

Par quantité équimolaire, on entend que chacune des trois lectines est utilisée en quantité identique par rapport aux autres. Il s'agit d'un ratio en poids de 1 :1 :1 entre les trois lectines.

Ainsi, selon un mode de réalisation, le présent texte concerne l'utilisation *in vitro* d'un mélange de trois lectines, choisi parmi les mélanges suivants : (UEA-1, ABA, ACA), (UEA-1, ABA, Jacaline), (UEA-1, ABA, GSL-I), (UEA-1, ABA, GSL-II), (UEA-1, ACA, Jacaline), (UEA-1, ACA, GSL-I), (UEA-1, ACA, GSL-II), (UEA-1, Jacaline, GSL-I), (UEA-1, Jacaline, GSL-II), (UEA-1, GSL-I, GSL-II), (ABA, ACA, Jacaline), (ABA, ACA, GSL-I), (ABA, ACA, GSL-II), (ABA, Jacaline, GSL-I), (ABA, Jacaline, GSL-II), (ABA, GSL-I, GSL-II), (ACA, Jacaline, GSL-I), (ACA, Jacaline, GSL-II), (ACA, GSL-I, GSL-II), (Jacaline, GSL-I, GSL-II), (TJA-II, ABA, ACA), (TJA-II, ABA, Jacaline), (TJA-II, ABA, GSL-I), (TJA-II, ABA, GSL-II), (TJA-II, ACA, Jacaline), (TJA-II, ACA, GSL-I), (TJA-II, ACA, GSL-II), (TJA-II, Jacaline, GSL-I), (TJA-II, Jacaline, GSL-II), (TJA-II, GSL-I, GSL-II), lesdites trois lectines étant en quantité équimolaire dans ledit mélange.

Selon un mode de réalisation, le présent texte concerne l'utilisation *in vitro* telle que précédemment décrite, de trois lectines, lesdites trois lectines étant choisies parmi un mélange de UEA-1 ou son homologue TJA-II, jacaline et ABA, ou un mélange de UEA-1 ou son homologue TJA-II, jacaline et ACA, et lesdites trois lectines étant présentes chacune dans le mélange en quantité équimolaire.

Selon un mode de réalisation, le présent texte concerne l'utilisation *in vitro* d'au moins trois lectines choisies parmi les lectines UEA-1 ou son homologue TJA-II, ABA, ACA, jacaline, GSL-I et GSL-II, lesdites au moins trois lectines étant en quantité non équimolaire.

Selon un mode de réalisation, le présent texte concerne l'utilisation *in vitro* d'un mélange de trois lectines choisies parmi les lectines UEA-1 ou son homologue TJA-II, ABA, ACA, jacaline, GSL-I et GSL-II, lesdites trois lectines étant en quantité non équimolaire dans ledit mélange.

Dans le cas de l'utilisation de trois lectines, par quantité non équimolaire, on entend que les trois lectines ne sont pas utilisées en quantité équimolaire les unes par rapport aux autres, et qu'au moins deux lectines sur les trois sont utilisées dans des quantités différentes. Il s'agit en particulier d'un ratio en poids 2 :1 :1 entre les trois lectines.

Ainsi, selon un mode de réalisation particulier, le présent texte concerne l'utilisation *in vitro* d'un mélange de trois lectines choisi parmi les mélanges suivants : (UEA-1, ABA, ACA), (UEA-1, ABA, Jacaline), (UEA-1, ABA, GSL-I), (UEA-1, ABA, GSL-II), (UEA-1, ACA, Jacaline), (UEA-1, ACA, GSL-I), (UEA-1, ACA, GSL-II), (UEA-1, Jacaline, GSL-I), (UEA-1, Jacaline, GSL-II), (UEA-1, GSL-I, GSL-II), (ABA, ACA, Jacaline), (ABA, ACA, GSL-I), (ABA, ACA, GSL-II), (ABA, Jacaline, GSL-I), (ABA, Jacaline, GSL-II), (ABA, GSL-I, GSL-II), (ACA, Jacaline, GSL-I), (ACA, Jacaline, GSL-II), (ACA, GSL-I, GSL-II), (Jacaline, GSL-I, GSL-II), (TJA-II, ABA, ACA), (TJA-II, ABA, Jacaline), (TJA-II, ABA, GSL-I), (TJA-II, ABA, GSL-II), (TJA-II, ACA, Jacaline), (TJA-II, ACA, GSL-I), (TJA-II, ACA, GSL-II), (TJA-II, Jacaline, GSL-I), (TJA-II, Jacaline, GSL-II), (TJA-II, GSL-I, GSL-II), lesdites trois lectines étant en quantité non équimolaire dans ledit mélange, en particulier dans un ratio en poids de 2 :1 :1, 1 :2 :1 ou 1 :1 :2.

Le présent texte décrit également un procédé de marquage *in vitro* des cellules souches cancéreuses d'organes impliqués dans la respiration, comprenant une étape de marquage des cellules souches cancéreuses d'organes impliqués dans la respiration avec au moins une lectine reconnaissant le motif fucose α 1-2 galactose particulière, pour obtenir des cellules souches cancéreuses d'organes impliqués dans la respiration marquées, dans un échantillon biologique.

Ainsi, le présent texte concerne également un procédé de marquage *in vitro* des cellules souches cancéreuses d'organes impliqués dans la respiration, comprenant une étape de marquage des cellules souches cancéreuses d'organes impliqués dans la respiration avec au moins une lectine choisie parmi les lectines UEA-1 ou son homologue TJA-II, ABA, ACA, jacaline, GSL-I et GSL-II, pour obtenir des cellules souches cancéreuses d'organes impliqués dans la respiration marquées, dans un échantillon biologique.

Ce procédé de marquage peut être intégré au sein d'un procédé de détection de cellules souches cancéreuses d'organes impliqués dans la respiration utilisant une lectine conjuguée à un marqueur choisi parmi un fluorophore, un radio-isotope, une enzyme, la biotine ou des billes d'or.

Le présent texte concerne également un procédé *in vitro* de détection de cellules souches cancéreuses d'organes impliqués dans la respiration, dans un échantillon biologique, comprenant :
(a) une étape de marquage des cellules souches cancéreuses d'organes impliqués dans la respiration avec au moins une lectine reconnaissant le motif fucose α 1-2 galactose particulière, ladite lectine étant conjuguée à un marqueur choisi parmi : un fluorophore, un radio-isotope, une enzyme, des billes d'or ou la biotine, pour obtenir un échantillon biologique dans lequel les cellules souches cancéreuses d'organes impliqués dans la respiration sont marquées par au moins une lectine, suivi d'
(b) une étape de détection desdites cellules souches cancéreuses d'organes impliqués dans la respiration marquées par au moins une lectine.

Ainsi, dans un mode de réalisation particulier, le présent texte concerne un procédé *in vitro* de détection tel que décrit ci-dessus, dans lequel ladite au moins une lectine est choisie parmi les lectines UEA-1 ou son homologue TJA-II, ABA, ACA, jacaline, GSL-I et GSL-II, notamment au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, notamment au moins trois lectines choisies parmi UEA-1 ou son homologue TJA-II, ABA, ACA, jacaline, GSL-I et GSL-II , en particulier le mélange de UEA-1, jacaline et ABA, ou le mélange de UEA-1, jacaline et ACA.

Dans un mode de réalisation particulier, le présent texte concerne un procédé *in vitro* de détection tel que décrit ci-dessus, dans lequel au moins deux lectines sont utilisées, lesdites au moins deux lectines étant en quantité équimolaire.

Dans un mode de réalisation particulier, le présent texte concerne un procédé *in vitro* de détection tel que décrit ci-dessus, dans lequel au moins deux lectines sont utilisées, lesdites au moins deux lectines étant en quantité non équimolaire

Dans un mode de réalisation particulier, le présent texte concerne un procédé *in vitro* de détection tel que décrit ci-dessus, dans lequel deux lectines sont utilisées, lesdites 2 lectines étant UEA-1 et GSL-I en quantité non équimolaire dans un ratio en poids de 2 :1, 3 :1 ou 4 :1.

Dans un mode de réalisation particulier, le présent texte concerne un procédé *in vitro* de détection tel que décrit ci-dessus, dans lequel ledit échantillon biologique est un échantillon biologique d'organes impliqués dans la respiration.

Dans un mode de réalisation particulier, le présent texte concerne un procédé *in vitro* de détection tel que décrit ci-dessus, dans lequel ledit organe impliqué dans la respiration est choisi parmi les poumons, le larynx, le pharynx, la bouche, le nez, la gorge, la langue, les sinus, la trachée et les glandes salivaires (les amygdales et la glande parotide).

Dans un mode de réalisation particulier, le présent texte concerne le procédé *in vitro* de détection de cellules souches cancéreuses pulmonaires, dans un échantillon biologique, comprenant:
(a) une étape de marquage des cellules souches cancéreuses pulmonaires avec au moins une lectine reconnaissant le motif fucose α 1-2 galactose, choisie parmi les lectines UEA-1, ABA, ACA, jacaline, GSL-I et GSL-II, ladite lectine étant conjuguée à un marqueur choisi parmi : un fluorophore, un radio-isotope, une enzyme, des billes d'or ou de la biotine, pour obtenir un échantillon biologique dans lequel les cellules souches cancéreuses pulmonaires sont marquées par au moins une lectine,
   suivi d'
(b) une étape de détection desdites cellules souches cancéreuses pulmonaires marquées par au moins une lectine.

Dans un mode de réalisation du procédé où la lectine est conjuguée à un fluorophore, les cellules souches cancéreuses d'organes impliqués dans la respiration marquées sont détectées par microscopie à fluorescence ou lecteur de fluorescence.

Dans un mode de réalisation du procédé où la lectine est conjuguée à un radio-isotope, les cellules souches cancéreuses d'organes impliqués dans la respiration marquées sont détectées par une gamma caméra.

Dans un mode de réalisation du procédé où la lectine est conjuguée à une enzyme catalysant la formation d'un produit coloré telle que la peroxydase de raifort (HRP), la phosphatase alcaline, la glucose oxydase ou la β-galactosidase, les cellules souches cancéreuses d'organes impliqués dans la respiration marquées sont détectées par microscopie UV/visible ou lecteur d'absorbance suite à l'ajout d'un substrat chromogène.

Dans un mode de réalisation du procédé où la lectine est conjuguée à une enzyme catalysant la formation d'un produit luminescent telle la HRP, les cellules souches cancéreuses d'organes impliqués dans la respiration marquées sont détectées en microscopie à luminescence ou par un lecteur de luminescence, suite à l'ajout d'un substrat chimioluminescent tel que le luminol.

Dans un mode de réalisation du procédé où la lectine est conjuguée à des billes d'or, les cellules souches cancéreuses d'organes impliqués dans la respiration marquées sont détectées par microscopie électronique.

Dans un mode de réalisation du procédé où la lectine est conjuguée avec de la biotine, lesdites cellules souches cancéreuses d'organes impliqués dans la respiration sont détectées par l'un des modes de détection décrit ci-dessus dans lequel ledit marqueur est conjugué à la streptavidine ou à l'avidine.

Le procédé de marquage peut également être intégré au sein d'un procédé d'isolement de cellules souches cancéreuses d'organes impliqués dans la respiration utilisant une lectine conjuguée un marqueur choisi parmi un fluorophore, un radio-isotope, une enzyme, la biotine ou des billes d'or.

Dans un mode de réalisation, le procédé selon la présente invention permet d'isoler les cellules souches cancéreuses d'organes impliqués dans la respiration. Cette étape d'isolement permet en particulier d'étudier les cellules souches cancéreuses d'organes impliqués dans la respiration détectées dans un échantillon tumoral d'organes impliqués dans la respiration afin, par exemple, de découvrir de nouveaux traitements capables d'éliminer ces cellules souches cancéreuses fréquemment à l'origine de récidives et de métastases.

Par « procédé *in vitro* d'isolement », on entend que l'échantillon biologique est enrichi en cellules souches cancéreuses (CSCs) d'organes impliqués dans la respiration par déplétion des cellules non souches cancéreuses (CNSCs) d'organes impliqués dans la respiration.

Les cellules souches cancéreuses d'organes impliqués dans la respiration sont spécifiquement séparées des autres types cellulaires présents dans l'échantillon, tel qu'éventuellement des cellules non souches cancéreuses d'organes impliqués dans la respiration (CNSCs), par l'utilisation d'au moins une lectine reconnaissant le motif fucose α 1-2 galactose particulière.

Cet enrichissement de l'échantillon en CSCs permet d'obtenir un échantillon biologique dans lequel les cellules souches cancéreuses d'organes impliqués dans la respiration sont majoritairement représentées, c'est-à-dire qu'elles sont présentes en quantité supérieure par rapport aux autres types cellulaires, notamment par rapport aux CNSCs.

Ainsi, la présente invention concerne également un procédé *in vitro* d'isolement de cellules souches cancéreuses d'organes impliqués dans la respiration, dans un échantillon biologique comprenant :
(a) une étape de marquage des cellules souches cancéreuses d'organes impliqués dans la respiration avec au moins une lectine reconnaissant le motif fucose α 1-2 galactose particulier, ladite lectine étant conjuguée à la biotine ou à un fluorophore, pour obtenir un échantillon biologique dans lequel les cellules souches cancéreuses d'organes impliqués dans la respiration sont marquées par au moins une lectine,
   suivi d'
(b) une étape d'isolement desdites cellules souches cancéreuses d'organes impliqués dans la respiration marquées par au moins une lectine, l'étape b) étant telle que définie dans la revendication 1.

Dans un mode particulier de réalisation, la présente invention concerne un procédé *in vitro* d'isolement de cellules souches cancéreuses d'organes impliqués dans la respiration, dans un échantillon comprenant :
(a) une étape de marquage des cellules souches cancéreuses d'organes impliqués dans la respiration avec au moins une lectine reconnaissant le motif fucose α 1-2 galactose particulière, ladite lectine étant conjuguée à la biotine, pour obtenir un échantillon biologique dans lequel les cellules souches cancéreuses d'organes impliqués dans la respiration sont marquées par au moins une lectine,
   suivi d'
(b) une étape d'isolement desdites cellules souches cancéreuses d'organes impliqués dans la respiration marquées par au moins une lectine, via un support fonctionnalisé avec de la streptavidine ou de l'avidine.

Cet isolement peut être suivi de la récupération de l'échantillon enrichi en cellules souches cancéreuses, par élimination du surnageant, puis par élution des cellules souches cancéreuses liées audit support.

Ladite élution peut être réalisée en condition acide pour rompre la liaison streptavidine/avidine - biotine.

Selon un mode de réalisation, ledit support est constitué par des billes magnétiques fonctionnalisées avec de la streptavidine ou de l'avidine et ladite étape d'isolement est réalisée par tri magnétique en présence d'un aimant.

Dans le cas particulier où ledit support est constitué par des billes magnétiques et où la streptavidine ou l'avidine est liée aux billes magnétiques par une liaison ADN, ladite élution est réalisée par traitement à la DNAse.

Dans un mode particulier de réalisation, la présente invention concerne un procédé *in vitro* d'isolement de cellules souches cancéreuses d'organes impliqués dans la respiration, dans un échantillon biologique comprenant :
(a) une étape de marquage des cellules souches cancéreuses d'organes impliqués dans la respiration avec au moins une lectine reconnaissant le motif fucose α 1-2 galactose particulière, ladite lectine étant conjuguée à un fluorophore pour obtenir un échantillon biologique dans lequel les cellules souches cancéreuses d'organes impliqués dans la respiration sont marquées par au moins une lectine,
   suivi d'
(b) une étape d'isolement desdites cellules souches cancéreuses d'organes impliqués dans la respiration marquées par au moins une lectine, par tri cellulaire en cytométrie en flux.

Dans un mode de réalisation particulier, la présente invention concerne également un procédé in vitro d'isolement tel que décrit ci-dessus, dans lequel ladite au moins une lectine est choisie parmi les lectines UEA-1 ou son homologue TJA-II, ABA, ACA, jacaline, GSL-I et GSL-II, notamment au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, notamment au moins trois lectines choisies parmi UEA-1 ou son homologue TJA-II, ABA, ACA, jacaline, GSL-I et GSL-II , en particulier le mélange de UEA-1, jacaline et ABA, ou le mélange de UEA-1, jacaline et ACA.

Dans un mode de réalisation particulier, la présente invention concerne également un procédé in vitro d'isolement tel que décrit ci-dessus, dans lequel au moins deux lectines sont utilisées, lesdites au moins deux lectines étant en quantité égale ou en quantité inégale

Dans un mode de réalisation particulier, la présente invention concerne également un procédé in vitro d'isolement tel que décrit ci-dessus, dans lequel au moins deux lectines sont utilisées, lesdites au moins deux lectines étant en quantité non équimolaire

Dans un mode de réalisation particulier, la présente invention concerne également un procédé in vitro d'isolement tel que décrit ci-dessus, dans lequel deux lectines sont utilisées, lesdites 2 lectines étant UEA-1 et GSL-I en quantité non équimolaire dans un ratio en poids de 2 :1, 3 :1 ou 4 :1

Dans un mode de réalisation particulier, la présente invention concerne également un procédé in vitro d'isolement tel que décrit ci-dessus, dans lequel ledit échantillon biologique est un échantillon biologique d'organes impliqués dans la respiration.

Dans la présente invention, ledit organe impliqué dans la respiration est choisi parmi les poumons, le larynx, le pharynx, la bouche, le nez, la gorge, la langue, les sinus, la trachée et les glandes salivaires (les amygdales et la glande parotide). Dans un mode de réalisation particulier, l'invention concerne un procédé *in vitro* d'isolement de cellules souches cancéreuses pulmonaires, dans un échantillon biologique comprenant :
(a) une étape de marquage des cellules souches cancéreuses pulmonaires avec au moins une lectine reconnaissant le motif fucose α 1-2 galactose, les lectines UEA-1, ABA, ACA, jacaline, GSL-I et GSL-II, ladite lectine étant conjuguée à la biotine ou à un fluorophore, pour obtenir un échantillon biologique dans lequel les cellules souches cancéreuses pulmonaires sont marquées par au moins une lectine,
   suivi d'
(b) une étape d'isolement desdites cellules souches cancéreuses pulmonaires marquées par au moins une lectine,

ladite étape d'isolement étant réalisée via un support fonctionnalisé avec de la streptavidine ou de l'avidine constitué par des billes magnétiques et en présence d'un aimant, lorsque ladite lectine est conjuguée à la biotine,
et ladite étape d'isolement étant réalisée par tri cellulaire en cytométrie en flux, lorsque ladite lectine est conjuguée à un fluorophore.

Le tri cellulaire en cytométrie en flux permet ainsi d'obtenir une fraction de l'échantillon enrichie en cellules souches cancéreuses.

Avant le marquage des cellules souches cancéreuses d'organes impliqués dans la respiration à l'étape (a), les cellules de l'échantillon sont avantageusement dissociées les unes des autres. Cette dissociation des cellules peut être réalisée selon des procédures conventionnelles, par exemple en utilisant une ou plusieurs enzymes capables de séparer les cellules les unes des autres sans altérer les glycanes exprimés à la surface des cellules, en particulier le motif fucose α 1-2 galactose particulier,. La dissociation des cellules peut par exemple être mise en œuvre avec le mélange Liberase^{®} commercialisé par la société Roche Diagnostic.

La présente invention concerne donc également des procédés selon l'invention, comprenant une étape préalable de dissociation des cellules de l'échantillon les unes des autres avant l'étape de marquage.

L'étude des cellules souches cancéreuses à des fins de recherche et de diagnostic représente aujourd'hui une nécessité, en particulier pour mettre en évidence de nouvelles substances capables d'agir contre ces cellules. L'étude de ces cellules est également particulièrement utile dans le cadre de la médecine personnalisée.

Les cellules souches cancéreuses d'organes impliqués dans la respiration sont une population de cellules particulières qui, en raison de leur résistance aux traitements de chimiothérapie, conduisent à la reformation de la tumeur et à la récidive tumorale. La présente invention permet donc, par la détection ou l'isolement des cellules souches cancéreuses d'organes impliqués dans la respiration, d'évaluer le risque de récidive du cancer d'organes impliqués dans la respiration. La détection des cellules souches cancéreuses d'organes impliqués dans la respiration, éventuellement suivie de leur quantification, permet d'évaluer les risques de progression d'une tumeur.

La présente invention concerne donc également l'utilisation d'une lectine reconnaissant le motif fucose α 1-2 galactose particulière, pour le diagnostic *in vitro* du risque de récidive et/ou de l'agressivité d'un cancer d'organes impliqués dans la respiration pour définir une valeur pronostique pour l'adaptation thérapeutique d'un cancer d'organes impliqués dans la respiration, utilisation telle que définie dans la revendication 6.

Ainsi, le présent texte concerne également une méthode de diagnostic *in vitro* du risque de récidive du cancer d'organes impliqués dans la respiration et/ou de l'agressivité du cancer d'organes impliqués dans la respiration pour définir une valeur pronostique pour l'adaptation thérapeutique d'un cancer d'organes impliqués dans la respiration, comprenant une étape de marquage des cellules souches cancéreuses d'organes impliqués dans la respiration d'un échantillon biologique d'organes impliqués dans la respiration avec au moins une lectine reconnaissant le motif fucose α 1-2 galactose particulière, pour obtenir des cellules souches cancéreuses d'organes impliqués dans la respiration marquées par au moins une lectine dans ledit échantillon.

Ainsi, le présent texte concerne également une méthode de diagnostic *in vitro* du risque de récidive du cancer d'organes impliqués dans la respiration et/ou de l'agressivité du cancer d'organes impliqués dans la respiration pour définir une valeur pronostique pour l'adaptation thérapeutique d'un cancer d'organes impliqués dans la respiration, comprenant une étape de marquage des cellules souches cancéreuses d'organes impliqués dans la respiration d'un échantillon biologique d'organes impliqués dans la respiration avec au moins une lectine choisie parmi les lectines UEA-1 ou son homologue TJA-II, ABA, ACA, jacaline, GSL-I et GSL-II, pour obtenir des cellules souches cancéreuses d'organes impliqués dans la respiration marquées par au moins une lectine dans ledit échantillon.

Selon un mode de réalisation particulier, la méthode de diagnostic comprend les étapes de :
(a) Marquage des cellules souches cancéreuses d'organes impliqués dans la respiration avec au moins une lectine reconnaissant le motif fucose α 1-2 galactose particulière, pour obtenir des cellules souches cancéreuses d'organes impliqués dans la respiration marquées par au moins une lectine, dans ledit échantillon biologique, ladite lectine étant conjuguée à un marqueur choisi parmi un fluorophore, un radio-isotope, une enzyme, des billes d'or ou de la biotine,
(b) Détection desdites cellules souches cancéreuses d'organes impliqués dans la respiration marquées par
   - microscopie en fluorescence ou lecteur de fluorescence lorsque la lectine est conjuguée à un fluorophore ou lorsque la lectine est conjuguée à la biotine et est détectée via un fluorophore conjugué à la streptavidine ou à l'avidine;
   - microscopie à luminescence ou lecteur de luminescence lorsque la lectine est conjuguée à une enzyme utilisant un substrat chimioluminescent ou lorsque la lectine est conjuguée à la biotine et est détectée via une enzyme utilisant un substrat chimioluminescent conjuguée à la streptavidine ou à l'avidine ;
   - gamma caméra lorsque la lectine est conjuguée à un radio-isotope, ou lorsque que la lectine est conjuguée à la biotine et est détectée via un radio-isotope conjugué à la streptavidine ou à l'avidine ;
   - microscopie UV/visible ou lecteur d'absorbance lorsque la lectine est conjuguée à une enzyme utilisant un substrat chromogène, ou lorsque la lectine est conjuguée à la biotine et est détectée via une enzyme utilisant un substrat chromogène conjuguée à la streptavidine ou à l'avidine ;
   - microscopie électronique lorsque la lectine est conjuguée à des billes d'or, ou lorsque la lectine est conjuguée à la biotine et est détectée via des billes d'or conjuguées à la streptavidine ou à l'avidine ;
(c) Eventuellement quantification des cellules souches cancéreuses d'organes impliqués dans la respiration ;
(d) Comparaison de l'intensité de la détection de cellules souches cancéreuses d'organes impliqués dans la respiration dans ledit échantillon biologique par rapport à l'intensité de la détection de cellules souches cancéreuses d'organes impliqués dans la respiration dans un échantillon sain jouxtant l'échantillon biologique,
   et éventuellement comparaison de la quantification de cellules souches cancéreuses d'organes impliqués dans la respiration dans ledit échantillon biologique par rapport à la quantification de cellules souches cancéreuses d'organes impliqués dans la respiration dans un échantillon sain jouxtant l'échantillon biologique
(e) Déduction du risque de récidive du cancer d'organes impliqués dans la respiration et/ou de l'agressivité du cancer d'organes impliqués dans la respiration pour définir une valeur pronostique pour l'adaptation thérapeutique d'un cancer d'organes impliqués dans la respiration à partir de la présence et éventuellement de la quantité de cellules souches cancéreuses d'organes impliqués dans la respiration.

Selon un mode de réalisation particulier, la méthode de diagnostic comprend les étapes de :
(a) Marquage des cellules souches cancéreuses d'organes impliqués dans la respiration avec au moins une lectine reconnaissant le motif fucose α 1-2 galactose particulière, pour obtenir des cellules souches cancéreuses d'organes impliqués dans la respiration marquées par au moins une lectine dans ledit échantillon biologique, ladite lectine étant conjuguée à un marqueur choisi parmi un fluorophore ou de la biotine,
(b) Isolement des cellules souches cancéreuses d'organes impliqués dans la respiration marquées par au moins une lectine conjuguée :
   - lors du marquage avec une lectine conjuguée la biotine, ledit isolement est effectué via un support fonctionnalisé avec de la streptavidine ou de l'avidine, en particulier ledit support fonctionnalisé est constitué de billes magnétiques fonctionnalisées avec de la streptavidine ou de l'avidine et ledit isolement est effectué par tri cellulaire magnétique en présence d'un aimant, ou
   - lors du marquage avec une lectine conjuguée un fluorophore, ledit isolement est effectué par tri cellulaire en cytométrie en flux.
(c) Nouveau marquage des cellules souches cancéreuses d'organes impliqués dans la respiration isolées avec au moins une lectine reconnaissant le motif fucose α 1-2 galactose particulière, pour obtenir des cellules souches cancéreuses d'organes impliqués dans la respiration isolées et marquées par le nouveau marquage,
   ladite lectine étant conjuguée à un marqueur choisi parmi un fluorophore, un radio-isotope, une enzyme, des billes d'or ou de la biotine,
(d) Détection desdites cellules souches cancéreuses d'organes impliqués dans la respiration isolées et marquées par le nouveau marquage par
   - microscopie en fluorescence ou lecteur de fluorescence lorsque la lectine est conjuguée à un fluorophore ou lorsque la lectine est conjuguée à la biotine et est détectée via un fluorophore conjugué à la streptavidine ou à l'avidine;
   - microscopie à luminescence ou lecteur de luminescence lorsque la lectine est conjuguée à une enzyme utilisant un substrat chimioluminescent ou lorsque la lectine est conjuguée à la biotine et est détectée via une enzyme utilisant un substrat chimioluminescent conjuguée à la streptavidine ou à l'avidine ;
   - gamma caméra lorsque la lectine est conjuguée à un radio-isotope, ou lorsque que la lectine est conjuguée à la biotine et est détectée via un radio-isotope conjugué à la streptavidine ou à l'avidine ;
   - microscopie UV/visible ou lecteur d'absorbance lorsque la lectine est conjuguée à une enzyme utilisant un substrat chromogène, ou lorsque la lectine est conjuguée à la biotine et est détectée via une enzyme utilisant un substrat chromogène conjuguée à la streptavidine ou à l'avidine ;
   - microscopie électronique lorsque la lectine est conjuguée à des billes d'or, ou lorsque la lectine est conjuguée à la biotine et est détectée via des billes d'or conjuguées à la streptavidine ou à l'avidine ;
(e) Eventuellement quantification des cellules souches cancéreuses d'organes impliqués dans la respiration ;
(f) Comparaison de l'intensité de la détection de cellules souches cancéreuses d'organes impliqués dans la respiration dans ledit échantillon biologique par rapport à l'intensité de la détection de cellules souches cancéreuses d'organes impliqués dans la respiration dans un échantillon sain jouxtant l'échantillon biologique,
   et éventuellement comparaison de la quantification de cellules souches cancéreuses d'organes impliqués dans la respiration dans ledit échantillon biologique par rapport à la quantification de cellules souches cancéreuses d'organes impliqués dans la respiration dans un échantillon sain jouxtant l'échantillon biologique ;
(g) Déduction du risque de récidive du cancer d'organes impliqués dans la respiration et/ou de l'agressivité du cancer d'organes impliqués dans la respiration pour définir une valeur pronostique pour l'adaptation thérapeutique d'un cancer d'organes impliqués dans la respiration à partir de la présence et éventuellement de la quantité de cellules souches cancéreuses d'organes impliqués dans la respiration.

Dans un mode de réalisation particulier, le présent texte concerne une méthode de diagnostic telle que décrite ci-dessus, dans laquelle ladite au moins une lectine est choisie parmi les lectines UEA-1 ou son homologue TJA-II, ABA, ACA, jacaline, GSL-I et GSL-II,
notamment au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I,
notamment au moins trois lectines choisies parmi UEA-1 ou son homologue TJA-II, ABA, ACA, jacaline, GSL-I et GSL-II , en particulier le mélange de UEA-1, jacaline et ABA, ou le mélange de UEA-1, jacaline et ACA.

Dans un mode de réalisation particulier, le présent texte concerne une méthode de diagnostic telle que décrite ci-dessus, dans laquelle au moins deux lectines sont utilisées, lesdites au moins deux lectines étant en quantité égale ou en quantité inégale.

Dans un mode de réalisation particulier, le présent texte concerne une méthode de diagnostic telle que décrite ci-dessus, dans laquelle au moins deux lectines sont utilisées, lesdites au moins deux lectines étant en quantité non équimolaire

Dans un mode de réalisation particulier, le présent texte concerne une méthode de diagnostic telle que décrite ci-dessus, dans laquelle deux lectines sont utilisées, lesdites 2 lectines étant UEA-1 et GSL-I en quantité non équimolaire dans un ratio en poids de 2 :1, 3 :1 ou 4 :1

Dans un mode de réalisation particulier, le présent texte concerne une méthode de diagnostic telle que décrite ci-dessus, dans laquelle ledit échantillon biologique est un échantillon biologique d'organes impliqués dans la respiration.

Dans un mode de réalisation particulier, le présent texte concerne une méthode de diagnostic telle que décrite ci-dessus, dans laquelle ledit organe impliqué dans la respiration est choisi parmi les poumons, le larynx, le pharynx, la bouche, le nez, la gorge, la langue, les sinus, la trachée et les glandes salivaires (les amygdales et la glande parotide).

Dans un mode de réalisation particulier, le présent texte concerne une méthode de diagnostic telle que décrite ci-dessus, dans laquelle ledit cancer est choisi parmi le cancer des poumons, le cancer du larynx, le cancer du pharynx, le cancer de la bouche, le cancer du nez, le cancer de la gorge, le cancer de la langue, le cancer des sinus, le cancer de la trachée et le cancer des glandes salivaires (c'est-à-dire le cancer des amygdales et/ou le cancer de la glande parotide).

Dans un mode de réalisation particulier, le présent texte concerne une méthode de diagnostic *in vitro* du risque de récidive du cancer pulmonaire et/ou de l'agressivité du cancer pulmonaire pour définir une valeur pronostique pour l'adaptation thérapeutique d'un cancer pulmonaire, comprenant une étape de marquage des cellules souches cancéreuses pulmonaires d'un échantillon biologique pulmonaire avec au moins une lectine reconnaissant le motif fucose α 1-2 galactose choisie parmi les lectines UEA-1, ABA, ACA, jacaline, GSL-I et GSL-II, pour obtenir des cellules souches cancéreuses pulmonaires marquées par au moins une lectine dans ledit échantillon.

Par le terme de « cancer pulmonaire », on entend cancer du poumon.

L'intensité de la détection des cellules souches cancéreuses d'organes impliqués dans la respiration, et éventuellement leur quantification est comparée par rapport un échantillon sain jouxtant l'échantillon biologique.

L'échantillon sain jouxtant l'échantillon biologique est utilisé comme témoin.

Par « échantillon sain jouxtant l'échantillon biologique » on entend un échantillon prélevé sur le même individu que l'échantillon biologique, mais dans un tissu voisin de celui où est prélevé ledit échantillon biologique, et qui ne présente pas de cellules tumorales en analyse d'anatomo-pathologie et qui ne présente pas de cellules souches cancéreuses par la méthode selon l'invention.

L'échantillon sain est donc un échantillon caractérisé par l'absence de cellules tumorales en analyse par anatomo-pathologie et l'absence de cellules souches cancéreuses par la méthode selon l'invention.

La quantification des cellules souches cancéreuses d'organes impliqués dans la respiration permet de déterminer l'agressivité du cancer d'organes impliqués dans la respiration. Cette quantification peut être établie par différentes méthodes telles que : la cytométrie en flux, le western blot, la PCR quantitative avec des marqueurs génériques tels que Oct-4, cMyc1, Gli-1 ou EpCam ou un test de clonogénicité.

Plusieurs de ces méthodes peuvent également être utilisées en parallèle afin de former un faisceau de présence de CSCs et ainsi augmenter la fiabilité de la quantification.

Dans un mode de réalisation particulier, la quantification des cellules souches cancéreuses d'organes impliqués dans la respiration est réalisée par un test de clonogénicité.

Un test de clonogénicité consiste en la mise en culture de l'échantillon biologique pour observer la capacité des cellules à reformer des sphères tumorales. Cette propriété d'auto-renouvellement et d'auto-réplication est propre aux cellules souches cancéreuses, une cellule souche cancéreuse unique est ainsi à l'origine d'une sphère tumorale formée. Ainsi, la comptabilisation des sphères tumorales formées, permet de quantifier les cellules souches cancéreuses dans l'échantillon.

Ces méthodes, avantageusement la qPCR avec des marqueurs génériques tels que Oct-4, c-Myc, Gli-1 ou EpCam et le test de clonogénicité, permettent également la détection des cellules souches cancéreuses afin de déterminer la présence ou l'absence de ces cellules après l'étape d'isolement des cellules souches cancéreuses.

Ces méthodes se présentent donc comme des alternatives aux susdites étapes (c) et (d), correspondant respectivement au nouveau marquage et à la détection des cellules souches cancéreuses d'organes impliqués dans la respiration isolées.

Cette détection est facilitée et rendue plus fiable de par l'enrichissement de l'échantillon en cellules souches cancéreuses.

La détection par ces méthodes permet également de valider l'efficacité de l'enrichissement de l'échantillon en cellules souches cancéreuses d'organes impliqués dans la respiration par la méthode selon l'invention, soit l'efficacité de la méthode à isoler des cellules souches cancéreuses d'organes impliqués dans la respiration.

Dans les méthodes de diagnostic, plus l'intensité de la détection dans l'échantillon biologique est élevée par rapport à un échantillon sain jouxtant le l'échantillon biologique, plus le risque de récidive du cancer d'organes impliqués dans la respiration est fort et plus le cancer est agressif.

De la même manière, plus la quantité de de cellules souches cancéreuses d'organes impliqués dans la respiration dans l'échantillon biologique est élevée par rapport à un échantillon sain jouxtant l'échantillon biologique, plus le risque de récidive du cancer d'organes impliqués dans la respiration est fort et plus le cancer est agressif.

La détection et la quantification des cellules souches cancéreuses d'organes impliqués dans la respiration dans un échantillon biologique permettent ainsi de déterminer l'agressivité du cancer d'organes impliqués dans la respiration et sa capacité à se développer.

La détection et la quantification des cellules souches cancéreuses d'organes impliqués dans la respiration s'inscrivent également dans une démarche de médecine personnalisée. En effet, la détection des cellules souches cancéreuses d'organes impliqués dans la respiration dans l'échantillon biologique permettant d'évaluer la valeur pronostique du traitement, permet ainsi d'adapter le traitement.

Comme déjà mentionné, la présente invention concerne également un kit de diagnostic *in vitro* du risque de récidive du cancer d'un organe impliqué dans la respiration et/ou de l'agressivité du cancer d'un organe impliqué dans la respiration pour définir une valeur pronostique pour l'adaptation thérapeutique d'un cancer d'un organe impliqué dans la respiration, dans lequel ledit organe impliqué dans la respiration est choisi parmi les poumons, le larynx, le pharynx, la bouche, le nez, la gorge, la langue, les sinus, la trachée et les glandes salivaires comprenant les amygdales et la glande parotide,
le kit comprenant au moins deux lectines choisies parmi le mélange de GSL-I et GSL-II ou le mélange UEA-1 et GSL-I, lesdites lectines étant conjuguées à la biotine, et des billes magnétiques fonctionnalisées avec de la streptavidine, et éventuellement au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, conjuguée à un fluorophore, un radio-isotope, une enzyme ou des billes d'or, ou
le kit comprenant au moins deux lectines choisies parmi le mélange de GSL-I et GSL-II ou le mélange UEA-1 et GSL-I, lesdites lectines étant conjuguées un fluorophore, et éventuellement au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, conjuguée à la biotine, un radio-isotope, une enzyme ou des billes d'or.

Le présente texte décrit aussi un kit de détection *in vitro* de cellules souches cancéreuses d'organes impliqués dans la respiration, dans un échantillon biologique, comprenant au moins une lectine reconnaissant le motif fucose α 1-2 galactose, , ladite lectine étant conjuguée à un marqueur choisi parmi : un fluorophore, un radio-isotope, une enzyme, des billes d'or ou de la biotine.

Le présente texte décrit également un kit d'isolement *in vitro* de cellules souches cancéreuses d'organes impliqués dans la respiration, dans un échantillon biologique, comprenant au moins une lectine reconnaissant le motif fucose α 1-2 galactose, ladite lectine étant conjuguée à la biotine, et des billes magnétiques fonctionnalisées avec de la streptavidine.

Le présente texte décrit également un kit d'isolement *in vitro* de cellules souches cancéreuses d'organes impliqués dans la respiration, dans un échantillon biologique comprenant au moins une lectine reconnaissant le motif fucose α 1-2 galactose, ladite lectine étant conjuguée à un fluorophore.

Les diagnostics selon l'invention sont réalisés sur un échantillon biologique, en particulier un échantillon d'organes impliqués dans la respiration.

Les kits décrits ci-dessus peuvent comprendre au moins deux lectines conjuguées choisies parmi les lectines UEA-1 ou son homologue TJA-II, ABA, ACA, jacaline, GSL-I et GSL-II, lesdites au moins deux lectines conjuguées étant en quantité équimolaire.

Selon un mode de réalisation, les kits peuvent comprendre deux lectines choisies parmi les lectines UEA-1 ou son homologue TJA-II, ABA, ACA, jacaline, GSL-I et GSL-II, lesdites deux lectines étant en quantité équimolaire.

Selon un mode de réalisation, les kits décrits peuvent comprendre deux lectines choisi parmi les mélanges suivants : (UEA-1, ABA), (UEA-1, ACA), (UEA-1, Jacaline), (UEA-1, GSL-I), (UEA-1, GSL-II), (ABA, ACA), (ABA, Jacaline), (ABA, GSL-I), (ABA, GSL-II), (ACA, Jacaline), (ACA, GSL-I), (ACA, GSL-II), (Jacaline, GSL-I), (Jacaline, GSL-II), (GSL-I, GSL-II), (TJA-II, ABA), (TJA-II, ACA), (TJA-II, Jacaline), (TJA-II, GSL-I), (TJA-II, GSL-II), lesdites deux lectines étant en quantité équimolaire.

Selon un mode de réalisation, les kits selon la présente invention peuvent comprendre deux lectines, lesdites deux lectines étant un mélange (GSL-I, GSL-II), dans lequel chacune des lectines sont en quantité équimolaire.

Selon un mode de réalisation, les kits selon la présente invention peuvent comprendre deux lectines, lesdites deux lectines étant un mélange (UEA-1, GSL-I), dans lequel chacune des lectines sont en quantité équimolaire.

Selon un mode de réalisation, les kits selon le présent texte peuvent comprendre au moins deux lectines choisies parmi les lectines UEA-1 ou son homologue TJA-II, ABA, ACA, jacaline, GSL-I et GSL-II, lesdites au moins deux lectines étant en quantité non équimolaire.

Selon un mode de réalisation, les kits selon le présent texte peuvent comprendre deux lectines choisies parmi les lectines UEA-1 ou son homologue TJA-II, ABA, ACA, jacaline, GSL-I et GSL-II lesdites deux lectines étant en quantité non équimolaire.

Selon un mode de réalisation, les kits selon le présent texte peuvent comprendre deux lectines choisi parmi les mélanges suivants : (UEA-1, ABA), (ABA, UEA-1), (UEA-1, ACA), (ACA, UEA-1), (UEA-1, Jacaline), (Jacaline, UEA-1), (UEA-1, GSL-I), (GSL-I, UEA-1), (UEA-1, GSL-II), (GSL-II, UEA-1), (ABA, ACA), (ACA, ABA), (ABA, Jacaline), (Jacaline, ABA), (ABA, GSL-I), (GSL-I, ABA), (ABA, GSL-II), (GSL-II, ABA), (ACA, Jacaline), (Jacaline, ABA), (ACA, GSL-I), (GSL-I, ACA), (ACA, GSL-II), (GSL-II, CA), (Jacaline, GSL-I), (GSL-I, Jacaline), (Jacaline, GSL-II), (GSL-II, Jacaline), (GSL-I, GSL-II), (GSL-II, GSL-I), (TJA-II, ABA), (ABA, TJA-II), (TJA-II, ACA), (ACA, TJA-II), (TJA-II, Jacaline), (Jacaline, TJA-II), (TJA-II, GSL-I), (GSL-I, TJA-II), (TJA-II, GSL-II), (GSL-II, TJA-II), lesdites deux lectines étant en quantité non équimolaire, en particulier dans un ratio en poids de 2 :1, 3 :1 ou 4 :1, plus particulièrement 2 :1.

Selon un mode de réalisation, les kits selon la présente invention peuvent comprendre deux lectines, lesdites deux lectines étant un mélange (UEA-1, GSL-I), dans lequel les lectines sont en quantité non équimolaire dans un ratio en poids de 2 : 1 soit 2 UEA-1 pour 1 GSL-I.

Selon un mode de réalisation, les kits selon la présente invention peuvent comprendre deux lectines, lesdites deux lectines étant un mélange (UEA-1, GSL-I), dans lequel les lectines sont en quantité non équimolaire dans un ratio en poids de 3 : 1 soit 3 UEA-1 pour 1 GSL-I.

Selon un mode de réalisation, les kits selon la présente invention peuvent comprendre deux lectines, lesdites deux lectines étant un mélange (UEA-1, GSL-I), dans lequel les lectines sont en quantité non équimolaire dans un ratio en poids de 4 : 1 soit 4 UEA-1 pour 1 GSL-I.

Les kits selon le présent texte décrits ci-dessus peuvent comprendre au moins trois lectines conjuguées choisies parmi les lectines UEA-1 ou son homologue TJA-II, ABA, ACA, jacaline, GSL-I et GSL-II, lesdites au moins deux lectines conjuguées étant en quantité équimolaire.

Selon un mode de réalisation, les kits selon le présent texte peuvent comprendre trois lectines choisies parmi les lectines UEA-1 ou son homologue TJA-II, ABA, ACA, jacaline, GSL-I et GSL-II, lesdites trois lectines étant chacune en quantité équimolaire.

Selon un mode de réalisation, les kits selon le présent texte peuvent comprendre trois lectines, choisi parmi les mélanges suivants : (UEA-1, ABA, ACA), (UEA-1, ABA, Jacaline), (UEA-1, ABA, GSL-I), (UEA-1, ABA, GSL-II), (UEA-1, ACA, Jacaline), (UEA-1, ACA, GSL-I), (UEA-1, ACA, GSL-II), (UEA-1, Jacaline, GSL-I), (UEA-1, Jacaline, GSL-II), (UEA-1, GSL-I, GSL-II), (ABA, ACA, Jacaline), (ABA, ACA, GSL-I), (ABA, ACA, GSL-II), (ABA, Jacaline, GSL-I), (ABA, Jacaline, GSL-II), (ABA, GSL-I, GSL-II), (ACA, Jacaline, GSL-I), (ACA, Jacaline, GSL-II), (ACA, GSL-I, GSL-II), (Jacaline, GSL-I, GSL-II), (TJA-II, ABA, ACA), (TJA-II, ABA, Jacaline), (TJA-II, ABA, GSL-I), (TJA-II, ABA, GSL-II), (TJA-II, ACA, Jacaline), (TJA-II, ACA, GSL-I), (TJA-II, ACA, GSL-II), (TJA-II, Jacaline, GSL-I), (TJA-II, Jacaline, GSL-II), (TJA-II, GSL-I, GSL-II), lesdites trois lectines étant en quantité équimolaire.

Selon un mode de réalisation, les kits selon le présent texte peuvent comprendre trois lectines, lesdites trois lectines étant choisies parmi un mélange de UEA-1 ou son homologue TJA-II, jacaline et ABA, ou un mélange de UEA-1 ou son homologue TJA-II, jacaline et ACA, et lesdites trois lectines étant présentes chacune dans le mélange en quantité équimolaire.

Selon un mode de réalisation, les kits selon le présent texte peuvent comprendre au moins trois lectines choisies parmi les lectines UEA-1 ou son homologue TJA-II, ABA, ACA, jacaline, GSL-I et GSL-II, lesdites au moins trois lectines étant en quantité non équimolaire.

Selon un mode de réalisation, les kits selon le présent texte peuvent comprendre trois lectines choisies parmi les lectines UEA-1 ou son homologue TJA-II, ABA, ACA, jacaline, GSL-I et GSL-II, lesdites trois lectines étant en quantité non équimolaire.

Selon un mode de réalisation, les kits selon le présent texte peuvent comprendre un mélange de trois lectines choisi parmi les mélanges suivants : (UEA-1, ABA, ACA), (UEA-1, ABA, Jacaline), (UEA-1, ABA, GSL-I), (UEA-1, ABA, GSL-II), (UEA-1, ACA, Jacaline), (UEA-1, ACA, GSL-I), (UEA-1, ACA, GSL-II), (UEA-1, Jacaline, GSL-I), (UEA-1, Jacaline, GSL-II), (UEA-1, GSL-I, GSL-II), (ABA, ACA, Jacaline), (ABA, ACA, GSL-I), (ABA, ACA, GSL-II), (ABA, Jacaline, GSL-I), (ABA, Jacaline, GSL-II), (ABA, GSL-I, GSL-II), (ACA, Jacaline, GSL-I), (ACA, Jacaline, GSL-II), (ACA, GSL-I, GSL-II), (Jacaline, GSL-I, GSL-II), (TJA-II, ABA, ACA), (TJA-II, ABA, Jacaline), (TJA-II, ABA, GSL-I), (TJA-II, ABA, GSL-II), (TJA-II, ACA, Jacaline), (TJA-II, ACA, GSL-I), (TJA-II, ACA, GSL-II), (TJA-II, Jacaline, GSL-I), (TJA-II, Jacaline, GSL-II), (TJA-II, GSL-I, GSL-II), lesdites trois lectines étant en quantité non équimolaire dans ledit mélange, en particulier dans un ratio en poids de 2 :1 :1, 1 :2 :1 ou 1 :1 :2.

Dans un autre aspect, le présent texte décrit une méthode de traitement d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses pouvant être réalisée avant, après ou simultanément à l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques pouvant être associée à une radiothérapie et/ou à une thérapie ciblée

On entend par « détection de la présence ou non de lésions tumorales », tout test permettant de détecter la présence ou non de lésions tumorales. Il peut notamment s'agir d'une analyse d'anatomo-pathologie classique consistant en une coloration HES (Hematoxyline Eosine Safran) permettant de définir la structuration des organes, un marquage de la protéine KI-67, permettant de déterminer un index de prolifération des cellules du tissus analysé ou encore un marquage de l'antigène carcino-embryonnaire (ACE), qui est un marqueur tumoral.

On entend par « étape de diagnostic de la présence ou non de cellules souches cancéreuses d'organes impliqués dans la respiration » une étape de mise en œuvre de la méthode de diagnostic selon la présente invention et telle que définie précédemment.

On entend par « thérapie ciblée », une thérapie sélective s'attaquant aux cellules cancéreuses en repérant chez elles une cible précise, telle qu'un récepteur, un gène ou une protéine. Les thérapies ciblées sont généralement utilisées en seconde ou en troisième ligne de traitement en cas d'échec thérapeutique. Dans le cas d'une thérapie ciblées, les médicaments préconisés sont des médicaments appartenant à la classe :
- des anti-VEGF, c'est-à-dire des agents ciblant le facteur de croissance de l'endothélium vasculaire
- des anti-EGF, c'est-à-dire des agents ciblant le facteur de croissance épidermique
- des anti-ALK, c'est-à-dire des agents ciblant la kinase « ALK » (Anaplastic Lymphoma Kinase)

Le présent texte décrit aussi une méthode de traitement d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses pouvant être réalisée avant, après ou simultanément à l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses pouvant être réalisée avant, après ou simultanément à l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques étant associée à une radiothérapie

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée après l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques pouvant être associée à une radiothérapie et/ou à une thérapie ciblée

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée après l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée après l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques étant associée à une radiothérapie

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée simultanément à l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques pouvant être associée à une radiothérapie et/ou à une thérapie ciblée

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée simultanément à l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée simultanément à l'étape a) de détection de la présence ou non de lésions tumorales
c) En cas de présence de cellules souches cancéreuses d'organes impliqués dans la respiration et de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques étant associée à une radiothérapie

Ledit agent chimio thérapeutique est choisi parmi la vinorelbine (Navelbine^{™}), le cisplatine, la gemcitabine (Gemzar^{™}), le carboplatine, le paclitaxel (Taxol^{™}), le docétaxel (Taxotère^{™}), le permetrexed (Alimta^{™}), l'erlotinib (Tarceva^{™}), le nivolumab, ou encore le bevacizumab (Avastin) ou une association deux à deux.

Dans un mode de réalisation particulier, les deux agents chimiothérapeutiques utilisés sont : la vinorelbine (Navelbine^{™}) et le cisplatine, la gemcitabine (Gemzar^{™}) et le cisplatine, la gemcitabine (Gemzar^{™}) et le carboplatine, le paclitaxel (Taxol^{™}) et le cisplatine, le paclitaxel (Taxol^{™}) et le carboplatine, le docétaxel (Taxotère^{™}) et le cisplatine, le docétaxel (Taxotère^{™}) et le carboplatine ou encore le permetrexed (Alimta^{™}) et le cisplatine.

Le présent texte décrit aussi une méthode de **prévention** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses pouvant être réalisée avant, après ou simultanément à l'étape a) de détection de la présence ou non de lésions tumorales
c) En cas de présence de cellules souches cancéreuses d'organes impliqués dans la respiration et d'absence de lésions tumorales, une étape de mise en place d'un suivi de l'évolution des cellules cancéreuses souches de l'organe impliqué dans la respiration détectées à l'étape a)

On entend par « suivi de l'évolution des cellules cancéreuses souches de l'organe impliqué dans la respiration », une nouvelle mise en œuvre de la méthode de prévention ci-dessus ayant permis la détection de cellules souches cancéreuses d'organes impliqués dans la respiration ou encore la réalisation d'une nouvelle biopsie accompagnée d'un nouveau marquage des cellules souches cancéreuses d'organes impliqués dans la respiration à l'aide des lectines reconnaissant spécifiquement le motif fucose α 1-2 galactose, plus particulièrement le motif fucose α 1-2 galactose β 1-4 N-acétylglucosamine selon la présente invention. Ce suivi sera effectué après les différents traitements par des agents chimio thérapeutiques, par radiothérapie et/ou par thérapie ciblée, tels que décrits précédemment, afin d'observer la régression ou non de la présence et/ou de la quantité des cellules souches cancéreuses de l'organe impliqué dans la respiration responsables de l'agressivité. Cette détection permettra d'orienter plus précisément le suivi et/ou le traitement à conduire, et ce de façon personnalisée pour le patient.

Le présent texte décrit aussi une méthode de **prévention** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration ladite étape b) de diagnostic de la présence ou non de cellules souches cancéreuses étant réalisée après l'étape a) de détection de la présence ou non de lésions tumorales
c) En cas de présence de cellules souches cancéreuses d'organes impliqués dans la respiration et d'absence de lésions tumorales, une étape de mise en place d'un suivi de l'évolution des cellules cancéreuses souches de l'organe impliqué dans la respiration détectées à l'étape a)

Le présent texte décrit aussi une méthode de **prévention** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée simultanément à l'étape a) de détection de la présence ou non de lésions tumorales
c) En cas de présence de cellules souches cancéreuses d'organes impliqués dans la respiration et d'absence de lésions tumorales, une étape de mise en place d'un suivi de l'évolution des cellules cancéreuses souches de l'organe impliqué dans la respiration détectées à l'étape a)

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses pouvant être réalisée avant, après ou simultanément à l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques pouvant être associée à une radiothérapie et/ou à une thérapie ciblée

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses pouvant être réalisée avant, après ou simultanément à l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses pouvant être réalisée avant, après ou simultanément à l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques étant associée à une radiothérapie

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée après l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques pouvant être associée à une radiothérapie et/ou à une thérapie ciblée

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée après l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée après l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques étant associée à une radiothérapie

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée simultanément à l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques pouvant être associée à une radiothérapie et/ou à une thérapie ciblée

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée simultanément à l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration

Le présent texte décrit aussi une méthode de **traitement** d'un cancer d'un organe impliqué dans la respiration comprenant :
a) Une étape de détection de la présence ou non de lésions tumorales dans un organe impliqué dans la respiration,
b) Une étape de diagnostic de la présence ou non de cellules souches cancéreuses d'organes impliqués dans la respiration
   ladite étape b) de diagnostic de la présence ou non de cellules souches cancéreuses étant réalisée simultanément à l'étape a) de détection de la présence ou non de lésions tumorales
c) En cas de présence de lésions tumorales, une étape d'administration d'un ou plusieurs agents chimio thérapeutiques pour le traitement du cancer d'un organe impliqué dans la respiration
   ladite administration d'un ou plusieurs agents chimio thérapeutiques étant associée à une radiothérapie

### DESCRIPTION DES FIGURES :

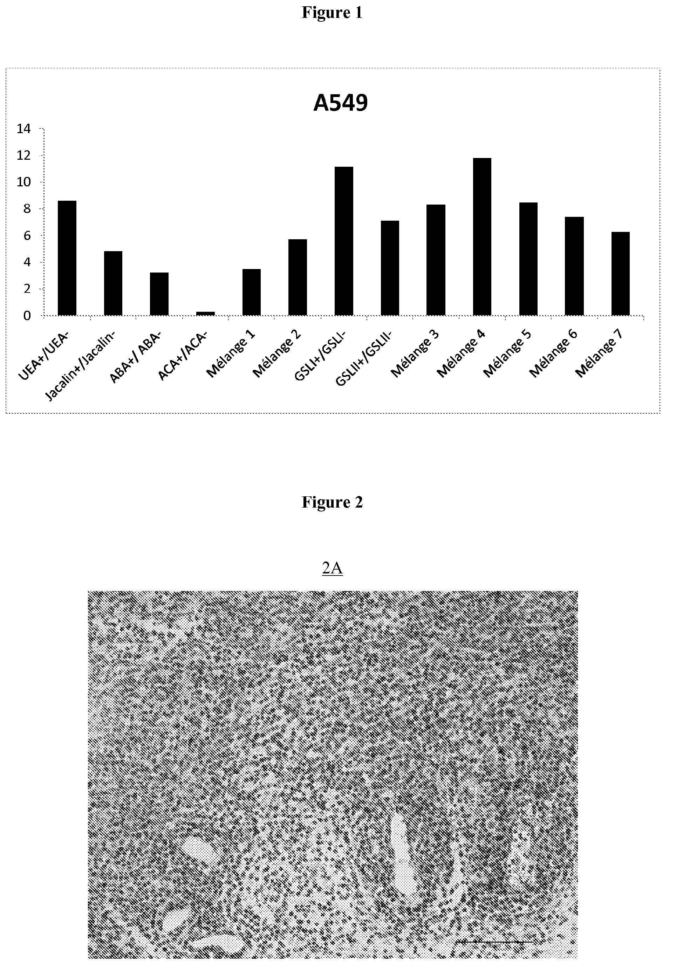

La **Figure 1** montre les résultats de la séparation de cellules souches cancéreuses pulmonaires, exemple phare du cancer des voies respiratoires, sur un échantillon de cellules issues de la lignée A549 et identifiées à l'aide du ratio Epcam High + / Epcam High - suite à l'utilisation des billes magnétiques sur lesquelles est greffée de la streptavidine et UEA-1 biotinylée (Lectine UEA-1), ABA biotinylée (Lectine ABA), ACA biotinylée (Lectine ACA), Jacaline biotinylée (Lectine Jacaline), GSL-I biotinylée (Lectine GSL-I), GSL-II biotinylée (Lectine GSL-II), le mélange de lectines biotinylées UEA-1 / Jacaline / ABA (Mélange 1 : UEA-1, Jacaline, ABA en quantité équimolaire), le mélange de lectines biotinylées UEA-1 / Jacaline / ACA (Mélange 2 : UEA-1, Jacaline, ACA en quantité équimolaire), le mélange de lectines biotinylées GSL-I / GSL-II (Mélange 3 : GSL-I et GSL-II en quantité équimolaire), le mélange de lectines biotinylées UEA-1 / GSL-I (Mélange 4 : UEA-1 et GSL-I en quantité équimolaire), le mélange de lectines biotinylées 2UEA-1 / GSL-I (Mélange 5 : UEA-1 et GSL-I en quantité non équimolaire, UEA-1 étant en quantité 2 fois supérieure à celle de GSL-I), le mélange de lectines biotinylées 3UEA-1 / GSL-I (Mélange 6: UEA-1 et GSL-I en quantité non équimolaire, UEA-1 étant en quantité 3 fois supérieure à celle de GSL-I) et enfin le mélange de lectines biotinylées 4UEA-1 / GSL-I (Mélange 7: UEA-1 et GSL-I en quantité non équimolaire, UEA-1 étant en quantité 4 fois supérieure à celle de GSL-I).

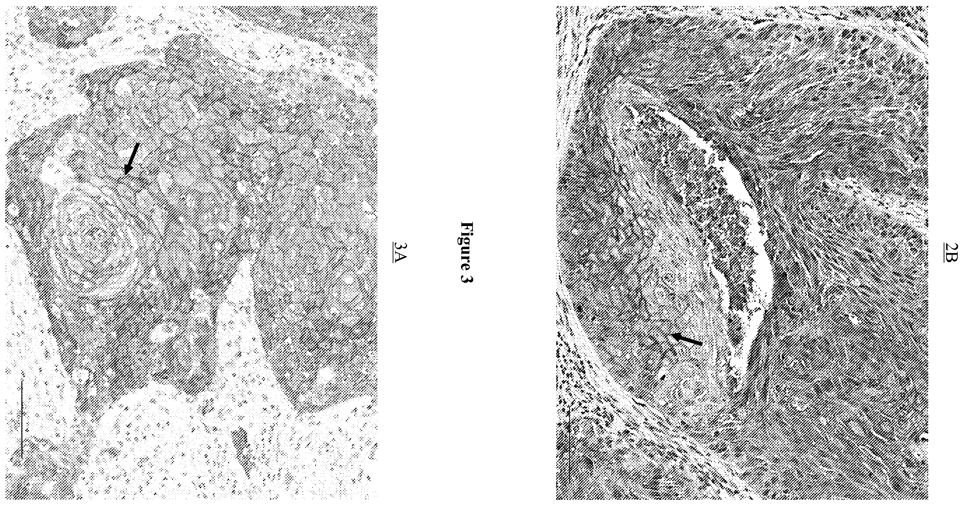

La **Figure 2** montre l'image de deux tissus tumoraux d'origine pulmonaire, exemple phare du cancer des voies respiratoires, marqué par le mélange UEA-1/ GSL-I en quantité équimolaire et provenant de deux patients différents (image du haut, notée 2A, correspondant à un premier patient et image du bas, notée 2B, correspondant à un second patient). L'image 2B représentant un tissu tumoral simplement marqué par le mélange de lectines UEA-1/ GSL-I présente un nombre important de cellules souches cancéreuses pulmonaires contourées par un marquage marron (couleur sombre contourant les cellules, voir flèche) marquant la présence de cette cible sucrée (2B). Au contraire, l'image 2A présente une parfaite négativité. Ainsi, un critère d'agressivité est défini de par la spécificité du marquage ciblant uniquement les cellules souches cancéreuses grâce au mélange de lectines UEA-1/ GSL-I sur des patients présentant à la base la même pathologie.

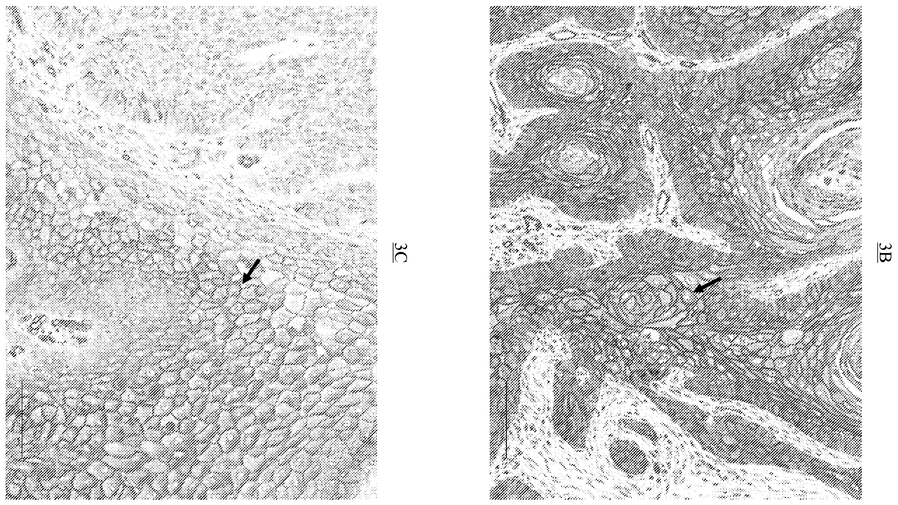

La **Figure 3** montre un panel de tissus appartenant aux voies respiratoires (A : langue, B : larynx, C : nez) marqué par le mélange UEA-1/ GSL-I en quantité équimolaire. Les images correspondantes des tissus tumoraux marqués par le mélange de lectines UEA-1/ GSL-I présentent un nombre important de cellules souches cancéreuses contourées par un marquage marron (couleur sombre contourant les cellules, voir flèche) marquant la présence de cellules souches cancéreuses. A l'image du poumon, un critère d'agressivité est donc défini de par la spécificité du marquage ciblant uniquement les cellules souches cancéreuses grâce au mélange de lectines UEA-1/ GSL-I en quantité équimolaire sur des patients atteints d'un cancer touchant les voies respiratoires.

### EXEMPLES

### Exemple 1 : Protocole d'isolement des cellules souches cancéreuses pulmonaires, exemple phare des cancers des voies respiratoires

### I. Matériels requis

### Réactifs et matériel

- Lectine individuelle biotinylée ou mélange de lectines biotinylées marquant spécifiquement les Cellules Souches Cancéreuses Pulmonaire (préparé à partir des lectines individuelles Vector Laboratories)
- Kit CELLection Biotin Binder (Invitrogen) contenant des billes magnétiques couplées à la streptavidine par une liaison ADN
- Aimant

### Tampons

- Versène (Invitrogen) comprenant du tampon phosphate salin (PBS) et de l'EDTA
- Tampon 1 : PBS (Tampon phosphate salin sans Ca²⁺ et Mg²⁺) avec 0.1% BSA (Albumine de sérum bovin), pH 7.4
- Tampon 2 : PBS (Phosphate Buffer Saline sans Ca²⁺ et Mg²⁺) avec 0.1% BSA (Bovine Serum Albumine) et 0.6% de citrate de sodium
- Tampon 3 : RPMI 1640 avec 1% de FCS (Sérum de veau fetal), 1mM CaCl₂ et 5mM de MgCl₂, pH 7.0-7.4.

### II. Durée de l'expérimentation

- 20 min pour préparer les cellules
- 20 min pour marquer les cellules
- 20 min pour incuber les cellules marquées avec les billes
- 10 min pour récupérer la suspension non enrichie en CSCs
- 15 min pour casser la liaison CSCs/billes
- 5 min pour récupérer la suspension enrichie en CSCs d'intérêt
- TOTAL : 1h30

### III. Mode opératoire par tri magnétique :

1. Préparation des cellules. Les cellules de la lignée A549 (lignée immortalisée de cancer du poumon provenant d'un prélèvement de patient atteint d'un cancer du poumon) sont décollées de leur support avec du Versène pendant 10 min à 37°C.
2. Les cellules sont comptées et le nombre de cellules est ajusté à 1.10⁷ dans l'échantillon.
3. La suspension cellulaire est centrifugée à 300g pendant 10 min puis le surnageant est éliminé.
4. Blocage des sites aspécifiques. 1mL de Tampon 2 est ajouté.
5. Marquage des cellules. Une quantité totale en lectines de 10 µg est ajoutée, de manière à ce que lors d'une pluralité de lectines, les quantités de chacune soient identiques.

Ainsi, sont ajoutés :
- 10 µg d'une lectine individuelle biotinylée choisie parmi : UEA-1, ABA, ACA, Jacaline, GSL-I et GSL-II, ou
- 3.33 µg de chaque lectine pour le mélange 1 (UEA-1 / ABA / Jacaline), ou
- 3.33 µg de chaque lectine pour le mélange 2 (UEA-1 / ACA / Jacaline) ou
- 5 µg de chaque lectine pour le mélange 3 (GSL-I / GSL-II), ou
- 5 µg de chaque lectine pour le mélange 4 (UEA-1 / GSL-I), ou
- 6,66 µg de UEA-1 et 3,33 µg de GSL-I pour le mélange 5, ou
- 7,5 µg de UEA-1 et 2,5 µg de GSL-I pour le mélange 6, ou
- 8 µg de UEA-1 et 2 µg de GSL-1 pour le mélange 7

Le mélange alors obtenu est incubé 10 min à 4°C.
6. 500µL de Tampon 2 est ajouté afin de laver les cellules et la suspension est centrifugée à 300g pendant 10 min et le surnageant est éliminé.
7. Ajout des billes. Les cellules sont resuspendues dans 1mL de Tampon 2 puis 25µL de billes magnétiques couplées à la streptavidine préalablement lavées sont ajoutées et resuspendues à l'aide de Tampon 1. Le mélange est incubé 20 min à 4°C sous agitation douce.
8. Récupération de la suspension NON enrichie en CSCs. Le tube est alors placé sur l'aimant pendant 2 min. Les cellules marquées par les lectine biotinylées et liées aux billes magnétiques couplées à la streptavidine, précipitent en direction de l'aimant (tri cellulaire magnétique) et sont alors séparées des cellules non marquées. Le surnageant contenant les cellules non marquées est ensuite retiré en maintenant le tube placé sur l'aimant, et est conservé dans un tube Falcon
9. Le tube contenant alors les cellules souches cancéreuses marquées est retiré de l'aimant, 1mL de Tampon 1 est ajouté, le tube est agité par vortex et replacé sur l'aimant pendant 2 min avant de conserver de nouveau le surnageant dans le même falcon qu'à l'étape 8. Cette étape est répétée deux fois.
10. Les cellules souches cancéreuses marquées, encore liées aux billes magnétiques, sont resuspendues à l'aide de 200µl de Tampon 3 préchauffé à 37°C. 4µL de tampon de coupure de la liaison cellules/billes constitué de DNaseI sont ajoutés. Ce mélange est incubé 15 min à température ambiante sous agitation douce.
11. La suspension est agitée avec une pipette vigoureusement 5 à 10 fois afin de faciliter la libération des cellules.
12. Récupération de la suspension enrichie en CSCs. Le tube est placé sur l'aimant pendant 2 min. Les billes magnétiques sont alors séparées des cellules souches cancéreuses marquées et le surnageant contenant les cellules souches cancéreuses marquées est transféré dans un tube contenant 200µL de tampon 3 préchauffé à 37°C. Les étapes 11 et 12 peuvent être répétées une nouvelle fois afin d'enrichir le rendement.

Ces expériences ont été réalisées dans des conditions similaires avec chacune des lectines biotinylées individuellement (UEA-1, ABA, ACA, Jacaline, GSL-I, GSL-II), des mélanges de deux lectines biotinylées (GSL-I / GSL-II = Mélange 3 ; UEA-1 / GSL-I = Mélange 4 ; 2UEA-1 / GSL-I = Mélange 5 ; 3UEA-1 / GSL-I = Mélange 6 ; 4UEA-1 / GSL-I = Mélange 7) ou de trois lectines lectines biotinylées (UEA-1 / ABA / Jacaline = Mélange 1 ; UEA-1 / ACA / Jacaline = Mélange 2).

Les résultats de ces différents essais sont présentés dans la figure 1. Il est à noter que l'exemple du poumon a été pris pour caractériser les cellules souches cancéreuses appartenant aux voies respiratoires. Ainsi que le montrent les résultats de ces essais, l'utilisation du mélange UEA1 / GSL-I en quantité équimolaire permet l'isolement des cellules souches cancéreuses pulmonaires et plus largement des voies respiratoires et ce de façon prépondérante.

De bons résultats sont également obtenus avec la lectine GSL II seule et la lectine UEA-1 seule et avec le mélange 3 (constitué d'un mélange des lectines GSL-I / GSL-II en quantité équimolaire), le mélange 5 (constitué d'un mélange des lectines UEA-1 et GSL-I en quantité non équimolaire 2 : 1) et le mélange 6 (constitué d'un mélange des lectines UEA-1 et GSL-I en quantité non équimolaire 3 :1).

L'isolement de cellules souches cancéreuses du larynx, du pharynx, de la bouche, du nez, de la gorge, de la langue, des sinus, de la trachée et des glandes salivaires (des amygdales et/ou de la glande parotide) a également été réalisé selon le protocole décrit dans l'exemple 1. Les résultats obtenus sont en accord avec ceux obtenus suite à l'isolement des cellules souches cancéreuses du poumon.

### Exemple 2 : Test de clonogénicité

L'objectif d'un test de clonogénicité est d'observer la capacité des cellules à reformer des sphères (correspondant chez le patient à la reformation d'une masse tumorale) donc leur capacité proliférative.

Le test de clonogénicité est dans cet exemple utilisé pour confirmer la présence des cellules souches cancéreuses pulmonaires et quantifier lesdites cellules dans un échantillon après isolement des cellules souches cancéreuses pulmonaires par la méthode d'isolement décrite dans la présente invention. Il permet ainsi de démontrer l'efficacité de la méthode d'isolement selon l'invention par rapport à un échantillon témoin non soumis cette méthode (cellules non triées).

Les tests de clonogénicité ont été réalisés dans une plaque à 6 puits à une densité de 500 cellules /cm² dans un milieu de composition DMEM (Gibco) supplémenté de 50 unités/mL de pénicilline, 50 unités/mL de streptomycine (Gibco) et 2.4 g/L de bicarbonate de sodium, 1 M de tampon HEPES (Sigma Aldrich, Saint-Quentin-Fallavier, France), 1X progestérone (Sigma Aldrich), 1X putrescine (Sigma), 0.025 g/mL héparine (Sigma Aldrich), 30% (m/v) glucose (Sigma Aldrich,), 1X supplément de croissance B27 (Invitrogen, Carlsbad, CA), 20 ng/mL EGF (Sigma Aldrich), 20 ng/mL FGF humain basique (Sigma Aldrich), 1X supplément insuline-transferrine- sélénite de sodium (Roche diagnostics, Meylan, France).

L'évolution des colonies a été observée après incubation à 37 °C dans une atmosphère de CO₂ pendant trois semaines et quantifiée avec le logiciel ImageJ^{®}.

Les cellules souches cancéreuses issues de cancers des organes impliqués dans la respiration sont isolées à partir de la méthode d'enrichissement décrite dans la présente invention conduisent à la formation de sphères contrairement au témoin. Il s'agit d'un test de clonogénicité ayant pour témoin des cellules non triées (T-), contre des cellules triées positivement par UEA-1 biotinylée (Lectine UEA-1), ABA biotinylée (Lectine ABA), ACA biotinylée (Lectine ACA), Jacaline biotinylée (Lectine Jacaline), GSL-I biotinylée (Lectine GSL-I), GSL-II biotinylée (Lectine GSL-II), le mélange de lectines biotinylées UEA-1 / Jacaline / ABA (Mélange 1 : UEA-1, Jacaline, ABA en quantité équimolaire), le mélange de lectines biotinylées UEA-1 / Jacaline / ACA (Mélange 2 : UEA-1, Jacaline, ACA en quantité équimolaire), et le mélange de lectines biotinylées GSL-I-GSL-II (Mélange 3 : GSL-I et GSL-II en quantité équimolaire), le mélange de lectines biotinylées UEA-1 / GSL-I (Mélange 4 : UEA-1 et GSL-I en quantité équimolaire), le mélange de lectines biotinylées 2UEA-1 / GSL-I (Mélange 5 : UEA-1 et GSL-I en quantité non équimolaire, UEA-1 étant en quantité 2 fois supérieure à celle de GSL-I), le mélange de lectines biotinylées 3UEA-1 / GSL-I (Mélange 6 : UEA-1 et GSL-I en quantité non équimolaire, UEA-1 étant en quantité 3 fois supérieure à celle de GSL-I) et enfin le mélange de lectines biotinylées 4UEA-1 / GSL-I (Mélange 7 : UEA-1 et GSL-I en quantité non équimolaire, UEA-1 étant en quantité 4 fois supérieure à celle de GSL-I). La méthode selon la présente invention permet donc d'obtenir des cellules souches capables de reformer des tumeurs (résultats non présentés).

### Exemple 3 : Marquage visible de lectines sur coupe histologique paraffinée : exemple du cancer pulmonaire

Matériel utilisé : Blocs de paraffine, Glace, Microtome, Lames superfrost^{®}, Automate Bond Max (Leica Microsystems) avec ordinateur, Consommables Leica (alcool, tampon de lavage, tampon ER1, tampon dewax, étiquettes, coverslips, tubes), tampon PBS-BSA 5%, Lectines biotinylées (UEA-1, Jacaline, ABA, ACA, GSL-I et GSL-II) (Vector Lab), Kit Bond Intense R detection (Leica), milieu de montage Leica, lamelles et microscope.

Les blocs de paraffine contenant les prélèvements de cancer pulmonaire provenant de chacun des patients identifiés par leur numéro (donné par le service d'anatomie pathologique) ont été placés dans la glace pendant environ 1 heure afin d'être refroidis, ceci afin de faciliter leur coupe au microtome à une épaisseur de 5µm.

Des lames dites « superfrost », ceci pour un maximum d'adhésion du tissu coupé ont été identifiées par les mêmes numéros que ceux présents sur les blocs. Une goutte d'eau a été placée au centre de chacune de ces lames.

Les coupes ont été réalisées au microtome et placées sur la goutte d'eau préalablement déposée. Les lames ont par la suite été posées sur une plaque chauffante à 37°C afin de faciliter leur adhésion et l'excédent d'eau a été retiré. L'ensemble des lames réalisé a été placé au sein d'une étuve à 37°C dans l'objectif de les sécher.

La suite de la manipulation a fait intervenir l'automate Bond Max de chez Leica relié à un ordinateur possédant un logiciel pilotant l'automate.

Durant le temps où les lames sont à l'étuve, l'ensemble de la manipulation de marquage immunohistochimique a été préparé en commençant par la vérification du niveau sur l'automate de chacun des produits nécessaires à la réalisation de la manipulation, puis à l'identification des lames avec leur même numéro ceci sur le logiciel pilotant l'automate. Des étiquettes permettant un protocole standardisé ont été générées. La dilution des lectines et leur quantité ont été calculées et le kit nécessaire préparé. Il est à noter que chacun des produits utilisés a dû être scanné et le niveau remis à zéro avant chacune des expérimentations effectuées.

Les étiquettes ont par la suite été collées sur leurs lames correspondantes à la sortie de l'étuve et des *coverslips,* éléments en plastique placés sur la coupe permettant une répartition homogène du produit sur toute la surface de la lame au cours de la manipulation grâce aux propriétés de contact, ont été placés sur chacune des lames.

Le portoir de lames a été placé dans l'automate et après reconnaissance par le lecteur de chacun des éléments et des lames identifiés par leurs codes-barres présents sur les étiquettes, la manipulation a été initiée.

Elle a commencé par un déparaffinage à la chaleur grâce au produit Dewax de chez Leica permettant par la suite l'accessibilité des anticorps. Cette étape ainsi que toutes les autres a été suivie de lavages, grâce au Bond Wash 10X préalablement dilué, ceci à trois reprises.

Cette étape a été suivie d'un prétraitement pendant 5 min avec un tampon citrate à pH =6 (le tampon ER1 de chez Leica), qui permet de démasquer les antigènes à atteindre dans le cadre de ce simple marquage, c'est-à-dire de les rendre accessibles.

La lectine biotinylée UEA-1 au 1/80^{ème} et la lectine biotynilée GSL-I au 1/200000^{ème} à l'aide du diluant PBS BSA-5% ont été placée de façon simultanée sur la coupe pendant 20 min.

Le kit Bond Intense R detection (Leica) grâce à l'intervention d'une streptavidine-HRP jouant le rôle d'anticorps secondaire a permis de par ses propriétés de révéler ces lectines biotinylées en marron grâce aux propriétés de la DAB, substrat de l'enzyme HRP (peroxydase du raifort), permettant de révéler le complexe biotine/streptavidine-HRP.

Une étape de contre coloration bleutée grâce à la présence d'hématoxyline a ensuite été réalisée pendant 7 min afin de rendre l'ensemble du prélèvement identifiable.

Les lames ont été retirées de l'automate. Les coupes ont été ensuite réhydratées en plongeant les lames manuellement dans un bain d'alcool par deux fois pendant 5 min. Cette étape de réhydratation a été poursuivie par un bain de toluène durant 5 min également.

Les lames ont pu dès lors être montées en mettant une goutte de milieu de montage (Leica). Les lames ont enfin été observées au microscope et des clichés ont été pris au grossissement 20.

Les résultats sont présentés dans les figures 2A et 2B.

Les résultats obtenus mettent en évidence la capacité des lectines utilisées dans l'invention à marquer sélectivement les cellules souches cancéreuses de tissus tumoraux d'origine pulmonaire.

Le simple marquage de deux tissus tumoraux pulmonaires par le mélange en quantité équimolaire de lectines UEA-1 / GSL-I biotinylées permet d'observer que des tissus bien que possédant des pathologies identiques peuvent présenter un critère d'agressivité bien différent.

En effet, la spécificité du marqueur utilisé dans l'invention ciblant les cellules souches cancéreuses est ici bien mise en valeur car elle montre une parfaite négativité dans le cadre de l'observation du tissu tumoral d'origine pulmonaire du premier patient (absence de couleur sombre entourant les cellules) montré à la figure 2A alors qu'une très forte positivité (couleur sombre contourant les cellules, voir flèche) est remarquée lors de l'observation du tissu tumoral d'origine pulmonaire du second patient (figure 2B). Une agressivité accrue (du fait de la présence de cellules souches cancéreuses) est à prendre en compte dans la prise en charge du patient.

### Exemple 4 : Marquage visible de lectines sur coupe histologique paraffinée : exemple du cancer du larynx

Cet exemple montre le marquage de tissus tumoraux de larynx par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux de larynx.

En effet, la figure 3B montre une très forte positivité (couleur sombre contourant les cellules, voir flèche) lors de l'observation du tissu tumoral de larynx. Une agressivité accrue (du fait de la présence de cellules souches cancéreuses) est à prendre en compte dans la prise en charge du patient.

### Exemple 5 : Marquage visible de lectines sur coupe histologique paraffinée : exemple du cancer du nez

Cet exemple montre le marquage de tissus tumoraux provenant du nez par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant du nez.

En effet, la figure 3C montre une très forte positivité (couleur sombre contourant les cellules, voir flèche) lors de l'observation du tissu tumoral provenant du nez. Une agressivité accrue (du fait de la présence de cellules souches cancéreuses) est à prendre en compte dans la prise en charge du patient.

### Exemple 6 : Marquage visible de lectines sur coupe histologique paraffinée : exemple du cancer de la langue

Cet exemple montre le marquage de tissus tumoraux provenant de la langue par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant de la langue.

En effet, la figure 3A montre une très forte positivité (couleur sombre contourant les cellules, voir flèche) lors de l'observation du tissu tumoral provenant de la langue. Une agressivité accrue (du fait de la présence de cellules souches cancéreuses) est à prendre en compte dans la prise en charge du patient.

### Exemple 7 : Marquage visible de lectines sur coupe histologique paraffinée : exemple du cancer pharynx

Cet exemple montre le marquage de tissus tumoraux provenant du pharynx par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant du pharynx.

### Exemple 8 : Marquage visible de lectines sur coupe histologique paraffinée : exemple du cancer de la bouche

Cet exemple montre le marquage de tissus tumoraux provenant de la bouche par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant de la bouche.

### Exemple 9 : Marquage visible de lectines sur coupe histologique paraffinée : exemple du cancer de la gorge

Cet exemple montre le marquage de tissus tumoraux provenant de la gorge par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant de la gorge.

### Exemple 10 : Marquage visible de lectines sur coupe histologique paraffinée : exemple du cancer de la trachée

Cet exemple montre le marquage de tissus tumoraux provenant de la trachée par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant de la trachée.

### Exemple 11 : Marquage visible de lectines sur coupe histologique paraffinée : exemple du cancer des sinus

Cet exemple montre le marquage de tissus tumoraux provenant des sinus par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant des sinus.

### Exemple 12 : Marquage visible de lectines sur coupe histologique paraffinée : exemple du cancer des amygdales (exemple de cancer des glandes salivaires)

Cet exemple montre le marquage de tissus tumoraux provenant des amygdales par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant des amygdales.

### Exemple 13 : Marquage visible de lectines sur coupe histologique paraffinée : exemple du cancer de la parotide (exemple de cancer des glandes salivaires)

Cet exemple montre le marquage de tissus tumoraux provenant de la parotide par le mélange UEA-1/ GSL-I en quantité équimolaire. Le marquage a été réalisé selon le protocole décrit dans l'exemple 3.

Les résultats obtenus mettent en évidence la capacité des lectines utilisées dans l'invention à marquer sélectivement les cellules souches cancéreuses de tissus tumoraux provenant de la parotide.

Les exemples 3 à 13 montrent que le simple marquage de tissus tumoraux appartenant aux voies respiratoires par le mélange en quantité équimolaire de lectines UEA-1 / GSL-I biotinylées permet d'observer que des tissus bien que possédant des pathologies identiques peuvent présenter un critère d'agressivité bien différent. Une agressivité accrue, dans ces cas présentant des pathologies cancéreuses variées mais appartenant aux voies respiratoires, est à prendre en compte dans la prise en charge du patient.

## Revendications

1. Procédé *in vitro* d'isolement de cellules souches cancéreuses d'organes impliqués dans la respiration, dans un échantillon biologique comprenant :
(a) une étape de marquage des cellules souches cancéreuses d'organes impliqués dans la respiration avec au moins une lectine choisie parmi les lectines UEA-1 ou son homologue TJA-II, ABA, ACA, jacaline, GSL-I et GSL-II, ladite lectine étant conjuguée à la biotine ou à un fluorophore, pour obtenir un échantillon biologique dans lequel les cellules souches cancéreuses d'organes impliqués dans la respiration sont marquées par au moins une lectine,
suivi d'
(b) une étape d'isolement desdites cellules souches cancéreuses d'organes impliqués dans la respiration marquées par au moins une lectine,
ladite étape d'isolement étant réalisée via un support fonctionnalisé avec de la streptavidine ou de l'avidine constitué par des billes magnétiques et en présence d'un aimant, lorsque ladite lectine est conjuguée à la biotine,
et ladite étape d'isolement étant réalisée par tri cellulaire en cytométrie en flux, lorsque ladite lectine est conjuguée à un fluorophore,
dans lequel lesdits organes impliqués dans la respiration sont choisis parmi les poumons, le larynx, le pharynx, la bouche, le nez, la gorge, la langue, les sinus, la trachée et les glandes salivaires comprenant les amygdales et la glande parotide.

2. Procédé *in vitro* d'isolement selon la revendication 1 dans lequel ladite étape d'isolement est suivie d'une étape de détection, ladite étape de détection comprenant :
(a) une étape de marquage des cellules souches cancéreuses d'organes impliqués dans la respiration avec au moins une lectine choisie parmi les lectines UEA-1 ou son homologue TJA-II, ABA, ACA, jacaline, GSL-I et GSL-II, ladite lectine étant conjuguée à un marqueur choisi parmi : un fluorophore, un radio-isotope, une enzyme, des billes d'or ou de la biotine, pour obtenir un échantillon biologique dans lequel les cellules souches cancéreuses d'organes impliqués dans la respiration sont marquées par au moins une lectine,
suivi d'
(b) une étape de détection desdites cellules souches cancéreuses d'organes impliqués dans la respiration marquées par au moins une lectine,
dans lequel lesdits organes impliqués dans la respiration sont choisi parmi les poumons, le larynx, le pharynx, la bouche, le nez, la gorge, la langue, les sinus, la trachée et les glandes salivaires comprenant les amygdales et la glande parotide.

3. Procédé *in vitro* d'isolement selon l'une quelconque des revendications 1 à 2, dans lequel ladite au moins une lectine est
au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I.

4. Procédé *in vitro* d'isolement selon l'une des revendications 1 à 3, dans lequel au moins deux lectines sont utilisées, lesdites au moins deux lectines étant en quantité égale ou en quantité inégale.

5. Procédé *in vitro* d'isolement selon l'une des revendications 1 à 3, dans lequel au moins deux lectines sont utilisées, lesdites au moins deux lectines étant en quantité non équimolaire,
lesdites 2 lectines étant de préférence UEA-1 et GSL-I en quantité non équimolaire dans un ratio en poids de 2 :1, 3 :1 ou 4 :1.

6. Utilisation d'au moins une lectine choisie parmi les lectines UEA-1 ou son homologue TJA-II, ABA, ACA, jacaline, GSL-I et GSL-II dans une méthode de diagnostic *in vitro* du risque de récidive du cancer d'un organe impliqué dans la respiration et/ou de l'agressivité du cancer d'un organe impliqué dans la respiration pour définir une valeur pronostique pour l'adaptation thérapeutique d'un cancer d'un organe impliqué dans la respiration,
dans lequel ledit organe impliqué dans la respiration est choisi parmi les poumons, le larynx, le pharynx, la bouche, le nez, la gorge, la langue, les sinus, la trachée et les glandes salivaires comprenant les amygdales et la glande parotide, dans laquelle la méthode de diagnostic comprend une étape de marquage des cellules souches cancéreuses d'organes impliqués dans la respiration d'un échantillon biologique d'organes impliqués dans la respiration avec au moins une lectine choisie parmi les lectines UEA-1 ou son homologue TJA-II, ABA, ACA, jacaline, GSL-I et GSL-II, pour obtenir des cellules souches cancéreuses d'organes impliqués dans la respiration marquées par au moins une lectine dans ledit échantillon.

7. Utilisation selon la revendication 6, dans laquelle la méthode de diagnostic comprend les étapes de :
(a) Marquage desdites cellules souches cancéreuses d'organes impliqués dans la respiration avec au moins une lectine choisie parmi les lectines UEA-1 ou son homologue TJA-II, ABA, ACA, jacaline, GSL-I et GSL-II, pour obtenir des cellules souches cancéreuses d'organes impliqués dans la respiration marquées par au moins une lectine dans ledit échantillon biologique,
ladite lectine étant conjuguée à un marqueur choisi parmi un fluorophore ou de la biotine,
(b) Isolement des cellules souches cancéreuses d'organes impliqués dans la respiration marquées par ladite au moins une lectine conjuguée :
- lors du marquage avec une lectine conjuguée la biotine, ledit isolement est effectué via un support fonctionnalisé avec de la streptavidine ou de l'avidine, en particulier ledit support fonctionnalisé est constitué de billes magnétiques fonctionnalisées avec de la streptavidine ou de l'avidine et ledit isolement est effectué par tri cellulaire magnétique en présence d'un aimant, ou
- lors du marquage avec une lectine conjuguée un fluorophore, ledit isolement est effectué par tri cellulaire en cytométrie en flux,
(c) Nouveau marquage des cellules souches cancéreuses d'organes impliqués dans la respiration isolées avec au moins une lectine reconnaissant choisie parmi les lectines UEA-1 ou son homologue TJA-II, ABA, ACA, jacaline, GSL-I et GSL-II, pour obtenir des cellules souches cancéreuses d'organes impliqués dans la respiration isolées et marquées,
ladite lectine étant conjuguée à un marqueur choisi parmi un fluorophore, un radio-isotope, une enzyme, des billes d'or ou de la biotine,
(d) Détection desdites cellules souches cancéreuses d'organes impliqués dans la respiration isolées et marquées par
- microscopie en fluorescence ou lecteur de fluorescence lorsque la lectine est conjuguée à un fluorophore, ou lorsque la lectine est conjuguée à la biotine et est détectée via un fluorophore conjugué à la streptavidine ou à l'avidine;
- microscopie à luminescence ou lecteur de luminescence lorsque la lectine est conjuguée à une enzyme utilisant un substrat chimioluminescent, ou lorsque la lectine est conjuguée à la biotine et est détectée via une enzyme utilisant un substrat chimioluminescent conjuguée à la streptavidine ou à l'avidine
- gamma caméra lorsque la lectine est conjuguée à un radio-isotope, ou lorsque la lectine est conjuguée à la biotine et est détectée via un radio-isotope conjugué à la streptavidine ou à l'avidine ;
- microscopie UV/visible ou lecteur d'absorbance lorsque la lectine est conjuguée à une enzyme utilisant un substrat chromogène, ou lorsque la lectine est conjuguée à la biotine et est détectée via une enzyme utilisant un substrat chromogène conjuguée à la streptavidine ou à l'avidine ;
- microscopie électronique lorsque la lectine est conjuguée à des billes d'or, ou lorsque la lectine est conjuguée à la biotine et est détectée via des billes d'or conjuguées à la streptavidine ou à l'avidine ;
(e) Eventuellement quantification des cellules souches cancéreuses d'organes impliqués dans la respiration;
(f) Comparaison de l'intensité de la détection de cellules souches cancéreuses d'organes impliqués dans la respiration dans ledit échantillon biologique par rapport à l'intensité de la détection de cellules souches cancéreuses d'organes impliqués dans la respiration dans un échantillon sain jouxtant l'échantillon biologique,
et éventuellement comparaison de la quantification de cellules souches cancéreuses d'organes impliqués dans la respiration dans ledit échantillon biologique par rapport à la quantification de cellules souches cancéreuses d'organes impliqués dans la respiration dans un échantillon sain jouxtant l'échantillon biologique,
(g) Déduction du risque de récidive du cancer d'un organe impliqué dans la respiration et/ou de l'agressivité du cancer d'un organe impliqué dans la respiration pour définir une valeur pronostique pour l'adaptation thérapeutique d'un cancer d'un organe impliqué dans la respiration à partir de la présence et éventuellement de la quantité de cellules souches cancéreuses d'organes impliqués dans la respiration.

8. Utilisation selon la revendication 6, dans laquelle la méthode de diagnostic selon la revendication 6, comprend les étapes de :
(a) Marquage desdites cellules souches cancéreuses d'organes impliqués dans la respiration avec au moins une lectine choisie parmi les lectines UEA-1 ou son homologue TJA-II, ABA, ACA, jacaline, GSL-I et GSL-II, pour obtenir des cellules souches cancéreuses d'organes impliqués dans la respiration marquées par au moins une lectine dans ledit échantillon biologique,
ladite lectine étant conjuguée à un marqueur choisi parmi un fluorophore, un radio-isotope, une enzyme, des billes d'or ou de la biotine,
(b) Détection desdites cellules souches cancéreuses d'organes impliqués dans la respiration marquées par
- microscopie en fluorescence ou lecteur de fluorescence lorsque la lectine est conjuguée à un fluorophore ou lorsque la lectine est conjuguée à la biotine et est détectée via un fluorophore conjugué à la streptavidine ou à l'avidine;
- microscopie à luminescence ou lecteur de luminescence lorsque la lectine est conjuguée à une enzyme utilisant un substrat chimioluminescent, ou lorsque la lectine est conjuguée à la biotine et est détectée via une enzyme utilisant un substrat chimioluminescent conjuguée à la streptavidine ou à l'avidine,
- gamma caméra lorsque la lectine est conjuguée à un radio-isotope, ou lorsque la lectine est conjuguée à la biotine et est détectée via un radio-isotope conjugué à la streptavidine ou à l'avidine ;
- microscopie UV/visible ou lecteur d'absorbance lorsque la lectine est conjuguée à une enzyme utilisant un substrat chromogène, ou lorsque la lectine est conjuguée à la biotine et est détectée via une enzyme utilisant un substrat chromogène conjuguée à la streptavidine ou à l'avidine ;
- microscopie électronique lorsque la lectine est conjuguée à des billes d'or, ou lorsque la lectine est conjuguée à la biotine et est détectée via des billes d'or conjuguées à la streptavidine ou à l'avidine ;
(c) Eventuellement quantification des cellules souches cancéreuses d'organes impliqués dans la respiration;
(d) Comparaison de l'intensité de la détection de cellules souches cancéreuses d'organes impliqués dans la respiration dans ledit échantillon biologique par rapport à l'intensité de la détection de cellules souches cancéreuses d'organes impliqués dans la respiration dans un échantillon sain jouxtant l'échantillon biologique,
et éventuellement comparaison de la quantification de cellules souches cancéreuses d'organes impliqués dans la respiration dans ledit échantillon biologique par rapport à la quantification de cellules souches cancéreuses d'organes impliqués dans la respiration dans un échantillon sain jouxtant l'échantillon biologique;
(e) Déduction du risque de récidive du cancer d'un organe impliqué dans la respiration et/ou de l'agressivité du cancer d'un organe impliqué dans la respiration pour définir une valeur pronostique pour l'adaptation thérapeutique d'un cancer d'un organe impliqué dans la respiration à partir de la présence et éventuellement de la quantité de cellules souches cancéreuses d'organes impliqués dans la respiration.

9. Utilisation selon l'une quelconque des revendications 6 à 8, dans laquelle ladite au moins une lectine est
au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I.

10. Utilisation selon l'une des revendications 6 à 9, dans laquelle au moins deux lectines sont utilisées, lesdites au moins deux lectines étant en quantité égale ou en quantité inégale ou en quantité non équimolaire,
lesdites 2 lectines étant de préférence UEA-1 et GSL-I en quantité non équimolaire dans un ratio en poids de 2 :1, 3 :1 ou 4 :1.

11. Kit de diagnostic *in vitro* du risque de récidive du cancer d'un organe impliqué dans la respiration et/ou de l'agressivité du cancer d'un organe impliqué dans la respiration pour définir une valeur pronostique pour l'adaptation thérapeutique d'un cancer d'un organe impliqué dans la respiration, dans lequel ledit organe impliqué dans la respiration est choisi parmi les poumons, le larynx, le pharynx, la bouche, le nez, la gorge, la langue, les sinus, la trachée et les glandes salivaires comprenant les amygdales et la glande parotide,
le kit comprenant au moins deux lectines choisies parmi le mélange de GSL-I et GSL-II ou le mélange UEA-1 et GSL-I, lesdites lectines étant conjuguées à la biotine, et des billes magnétiques fonctionnalisées avec de la streptavidine, et éventuellement au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, conjuguées à un fluorophore, un radio-isotope, une enzyme ou des billes d'or, ou
le kit comprenant au moins deux lectines choisies parmi le mélange de GSL-I et GSL-II ou le mélange UEA-1 et GSL-I, lesdites lectines étant conjuguées à un fluorophore, et éventuellement au moins deux lectines choisies parmi UEA-1 ou son homologue TJA-II, ABA, ACA, jacaline, GSL-I et GSL-II, en particulier le mélange de GSL-I et GSL-II ou le mélange UEA-1 et GSL-I, conjuguée à la biotine, un radio-isotope, une enzyme ou des billes d'or.

12. Kit de diagnostic selon la revendication 11, lesdites au moins deux lectines étant en quantité égale ou en quantité inégale.

13. Kit de diagnostic selon l'une des revendications 11 à 12, lesdites au moins deux lectines étant en quantité non équimolaire, lesdites 2 lectines étant de préférence UEA-1 et GSL-I en quantité non équimolaire dans un ratio en poids de 2 :1,3 :1 ou 4 :1.

## Patentansprüche

1. *In-vitro*-Verfahren zur Isolierung von Krebsstammzellen von an der Atmung beteiligten Organen in einer biologischen Probe, umfassend:
(a) einen Schritt des Markierens der Krebsstammzellen von an der Atmung beteiligten Organen mit mindestens einem Lektin ausgewählt aus UEA-1-Lektinen oder seinem Homologen TJA-II, ABA, ACA, Jakalin, GSL-I und GSL-II, wobei das Lektin mit Biotin oder einem Fluorophor konjugiert ist, um eine biologische Probe zu erhalten, in der die Krebsstammzellen von an der Atmung beteiligten Organen mit mindestens einem Lektin markiert sind,
und anschließend
(b) einen Schritt der Isolierung der Krebsstammzellen von an der Atmung beteiligten Organen, die mit mindestens einem Lektin markiert sind,
wobei der Isolierungsschritt über einen mit Streptavidin oder Avidin funktionalisierten, aus Magnetkugeln bestehenden Träger mithilfe eines Magneten durchgeführt wird, wenn das Lektin mit Biotin konjugiert ist,
und wobei der Isolierungsschritt durch Durchflusszytometrie-Zellsortierung durchgeführt wird, wenn das Lektin mit einem Fluorophor konjugiert ist,
wobei die an der Atmung beteiligten Organe ausgewählt sind aus Lunge, Kehlkopf, Rachen, Mund, Nase, Kehle, Zunge, Nebenhöhlen, Luftröhre und Speicheldrüsen, einschließlich Mandeln und Ohrspeicheldrüse.

2. *In-vitro*-Isolierungsverfahren nach Anspruch 1, wobei auf den Isolierungsschritt ein Nachweisschritt folgt, wobei der Nachweisschritt umfasst:
(a) einen Schritt des Markierens der Krebsstammzellen von an der Atmung beteiligten Organen mit mindestens einem Lektin ausgewählt aus UEA-1-Lektinen oder seinem Homologen TJA-II, ABA, ACA, Jakalin, GSL-I und GSL-II, wobei das Lektin mit einem Marker konjugiert ist, der ausgewählt ist aus: einem Fluorophor, einem Radioisotop, einem Enzym, Goldkugeln oder Biotin, um eine biologische Probe zu erhalten, in der die Krebsstammzellen von an der Atmung beteiligten Organen mit mindestens einem Lektin markiert sind,
und anschließend
(b) einen Schritt des Nachweises der Krebsstammzellen von an der Atmung beteiligten Organen, die mit mindestens einem Lektin markiert sind,
wobei die an der Atmung beteiligten Organe ausgewählt sind aus Lunge, Kehlkopf, Rachen, Mund, Nase, Kehle, Zunge, Nebenhöhlen, Luftröhre und Speicheldrüsen, einschließlich Mandeln und Ohrspeicheldrüse.

3. *In-vitro*-Isolierungsverfahren nach einem der Ansprüche 1 bis 2, wobei das mindestens eine Lektin
mindestens zwei Lektine ist, ausgewählt aus UEA-1 oder seinem Homologen TJA-II, ABA, ACA, Jakalin, GSL-I und GSL-II, insbesondere die Mischung aus GSL-I und GSL-II oder die Mischung aus UEA-1 und GSL-I.

4. *In-vitro*-Isolierungsverfahren nach einem der Ansprüche 1 bis 3, wobei mindestens zwei Lektine verwendet werden, wobei die mindestens zwei Lektine in gleicher oder ungleicher Menge sind.

5. *In-vitro*-Isolierungsverfahren nach einem der Ansprüche 1 bis 3, wobei mindestens zwei Lektine verwendet werden, wobei die mindestens zwei Lektine in nicht äquimolarer Menge sind,
wobei die 2 Lektine vorzugsweise UEA-1 und GSL-I in nicht äquimolarer Menge in einem Gewichtsverhältnis von 2:1, 3:1 oder 4:1 sind.

6. Verwendung von mindestens einem Lektin, ausgewählt aus UEA-1-Lektinen oder seinem Homologen TJA-II, ABA, ACA, Jakalin, GSL-I und GSL-II in einem *In-vitro-*Diagnoseverfahren für das Rückfallrisiko des Krebses eines an der Atmung beteiligten Organs und/oder der Aggressivität des Krebses eines an der Atmung beteiligten Organs, um einen prognostischen Wert für die therapeutische Anpassung eines Krebses eines an der Atmung beteiligten Organs zu definieren,
wobei das an der Atmung beteiligte Organ ausgewählt ist aus Lunge, Kehlkopf, Rachen, Mund, Nase, Kehle, Zunge, Nebenhöhlen, Luftröhre und Speicheldrüsen, einschließlich Mandeln und Ohrspeicheldrüse, wobei das Diagnoseverfahren einen Schritt des Markierens der Krebsstammzellen von an der Atmung beteiligten Organen aus einer biologischen Probe von an der Atmung beteiligten Organen mit mindestens einem Lektin ausgewählt aus UEA-1-Lektinen oder seinem Homologen TJA-II, ABA, ACA, Jakalin, GSL-I und GSL-II umfasst, um Krebsstammzellen von an der Atmung beteiligten Organen zu erhalten, die in der Probe durch mindestens ein Lektin markiert sind.

7. Verwendung nach Anspruch 6, wobei das Diagnoseverfahren folgende Schritte umfasst:
(a) Markieren der Krebsstammzellen von an der Atmung beteiligten Organen mit mindestens einem Lektin, ausgewählt aus UEA-1-Lektinen oder seinem Homologen TJA-II, ABA, ACA, Jakalin, GSL-I und GSL-II, um Krebsstammzellen von an der Atmung beteiligten Organen zu erhalten, die in der biologischen Probe durch mindestens ein Lektin markiert sind,
wobei das Lektin mit einem Marker konjugiert ist, der aus einem Fluorophor oder Biotin ausgewählt ist,
(b) Isolierung der durch mindestens ein konjugiertes Lektin markierten Krebsstammzellen von an der Atmung beteiligten Organen:
- bei der Markierung mit einem mit Biotin konjugierten Lektin erfolgt die Isolierung über einen mit Streptavidin oder Avidin funktionalisierten Träger, insbesondere besteht der funktionalisierte Träger aus mit Streptavidin oder Avidin funktionalisierten Magnetkugeln und die Isolierung erfolgt durch magnetische Zellsortierung mithilfe eines Magneten, oder
- bei der Markierung mit einem Lektin, das mit einem Fluorophor konjugiert ist, erfolgt die Isolierung durch Durchflusszytometrie-Zellsortierung,
(c) Neues Markieren der isolierten Krebsstammzellen von an der Atmung beteiligten Organen mit mindestens einem erkennenden Lektin, ausgewählt aus UEA-1-Lektinen oder seinem Homologen TJA-II, ABA, ACA, Jakalin, GSL-I und GSL-II, um isolierte und markierte Krebsstammzellen von an der Atmung beteiligten Organen zu erhalten,
wobei das Lektin mit einem Marker konjugiert ist, der aus einem Fluorophor, einem Radioisotop, einem Enzym, Goldkugeln oder Biotin ausgewählt ist,
(d) Nachweis der isolierten und markierten Krebsstammzellen von an der Atmung beteiligten Organen durch
- Fluoreszenzmikroskopie oder Fluoreszenzscanner, wenn das Lektin mit einem Fluorophor konjugiert ist, oder wenn das Lektin mit Biotin konjugiert ist und über ein mit Streptavidin oder Avidin konjugiertes Fluorophor nachgewiesen wird;
- Lumineszenzmikroskopie oder Lumineszenzscanner, wenn das Lektin mit einem Enzym konjugiert ist, das ein chemilumineszierendes Substrat verwendet, oder wenn Lektin mit Biotin konjugiert ist und über ein Enzym nachgewiesen wird, das ein chemilumineszierendes Substrat verwendet, das mit Streptavidin oder Avidin konjugiert ist
- Gammakamera, wenn das Lektin mit einem Radioisotop konjugiert ist oder wenn das Lektin mit Biotin konjugiert ist und über ein mit Streptavidin oder Avidin konjugiertes Radioisotop nachgewiesen wird;
- UV/VIS-Mikroskopie oder Absorptionsscanner, wenn das Lektin mit einem Enzym konjugiert ist, das ein chromogenes Substrat verwendet, oder wenn das Lektin mit Biotin konjugiert ist und über ein Enzym nachgewiesen wird, das ein chromogenes Substrat verwendet, das mit Streptavidin oder Avidin konjugiert ist;
- Elektronenmikroskopie, wenn das Lektin mit Goldkugeln konjugiert ist, oder wenn das Lektin mit Biotin konjugiert ist und über mit mit Streptavidin oder Avidin konjugierten Goldkugeln nachgewiesen wird;
(e) Ggf. Quantifizierung der Krebsstammzellen von an der Atmung beteiligten Organen;
(f) Vergleich der Intensität des Nachweises von Krebsstammzellen von an der Atmung beteiligten Organen in der biologischen Probe mit der Intensität des Nachweises von Krebsstammzellen von an der Atmung beteiligten Organen in einer gesunden Probe neben der biologischen Probe,
und gegebenenfalls Vergleich der Quantifizierung von Krebsstammzellen von an der Atmung beteiligten Organen in der biologischen Probe mit der Quantifizierung von Krebsstammzellen von an der Atmung beteiligten Organen in einer gesunden Probe neben der biologischen Probe,
(g) Ableitung des Rückfallrisikos des Krebses eines an der Atmung beteiligten Organs und/oder der Aggressivität eines Krebses eines an der Atmung beteiligten Organs, um einen prognostischen Wert für die therapeutische Anpassung eines Krebses eines an der Atmung beteiligten Organs aus dem Vorhandensein und gegebenenfalls der Menge von Krebsstammzellen von an der Atmung beteiligten Organen zu definieren.

8. Verwendung nach Anspruch 6, wobei das Diagnoseverfahren nach Anspruch 6 folgende Schritte umfasst:
(a) Markieren der Krebsstammzellen von an der Atmung beteiligten Organen mit mindestens einem Lektin, ausgewählt aus UEA-1-Lektinen oder seinem Homologen TJA-II, ABA, ACA, Jakalin, GSL-I und GSL-II, um Krebsstammzellen von an der Atmung beteiligten Organen zu erhalten, die in der biologischen Probe durch mindestens ein Lektin markiert sind,
wobei das Lektin mit einem Marker konjugiert ist, der aus einem Fluorophor, einem Radioisotop, einem Enzym, Goldkugeln oder Biotin ausgewählt ist,
(b) Nachweis der markierten Krebsstammzellen von an der Atmung beteiligten Organen durch
- Fluoreszenzmikroskopie oder Fluoreszenzscanner, wenn das Lektin mit einem Fluorophor konjugiert ist, oder wenn das Lektin mit Biotin konjugiert ist und über ein mit Streptavidin oder Avidin konjugiertes Fluorophor nachgewiesen wird;
- Lumineszenzmikroskopie oder Lumineszenzscanner, wenn das Lektin mit einem Enzym konjugiert ist, das ein chemilumineszierendes Substrat verwendet, oder wenn Lektin mit Biotin konjugiert ist und über ein Enzym nachgewiesen wird, das ein chemilumineszierendes Substrat verwendet, das mit Streptavidin oder Avidin konjugiert ist,
- Gammakamera, wenn das Lektin mit einem Radioisotop konjugiert ist oder wenn das Lektin mit Biotin konjugiert ist und über ein mit Streptavidin oder Avidin konjugiertes Radioisotop nachgewiesen wird;
- UV/VIS-Mikroskopie oder Absorptionsscanner, wenn das Lektin mit einem Enzym konjugiert ist, das ein chromogenes Substrat verwendet, oder wenn das Lektin mit Biotin konjugiert ist und über ein Enzym nachgewiesen wird, das ein chromogenes Substrat verwendet, das mit Streptavidin oder Avidin konjugiert ist;
- Elektronenmikroskopie, wenn das Lektin mit Goldkugeln konjugiert ist, oder wenn das Lektin mit Biotin konjugiert ist und über mit Streptavidin oder Avidin konjugierten Goldkugeln nachgewiesen wird;
(c) Ggf. Quantifizierung der Krebsstammzellen von an der Atmung beteiligten Organen;
(d) Vergleich der Intensität des Nachweises von Krebsstammzellen von an der Atmung beteiligten Organen in der biologischen Probe mit der Intensität des Nachweises von Krebsstammzellen von an der Atmung beteiligten Organen in einer gesunden Probe neben der biologischen Probe,
und gegebenenfalls Vergleich der Quantifizierung von Krebsstammzellen von an der Atmung beteiligten Organen in der biologischen Probe mit der Quantifizierung von Krebsstammzellen von an der Atmung beteiligten Organen in einer gesunden Probe neben der biologischen Probe;
(e) Ableitung des Rückfallrisikos des Krebses eines an der Atmung beteiligten Organs und/oder der Aggressivität eines Krebses eines an der Atmung beteiligten Organs, um einen prognostischen Wert für die therapeutische Anpassung eines Krebses eines an der Atmung beteiligten Organs aus dem Vorhandensein und gegebenenfalls der Menge von Krebsstammzellen von an der Atmung beteiligten Organen zu definieren.

9. Verwendung nach einem der Ansprüche 6 bis 8, wobei das mindestens eine Lektin mindestens zwei Lektine ist, ausgewählt aus UEA-1 oder seinem Homologen TJA-II, ABA, ACA, Jakalin, GSL-I und GSL-II, insbesondere die Mischung aus GSL-I und GSL-II oder die Mischung aus UEA-1 und GSL-I.

10. Verwendung nach einem der Ansprüche 6 bis 9, wobei mindestens zwei Lektine verwendet werden, wobei die mindestens zwei Lektine in gleicher oder ungleicher Menge oder in nicht äquimolarer Menge sind,
wobei die 2 Lektine vorzugsweise UEA-1 und GSL-I in nicht äquimolarer Menge in einem Gewichtsverhältnis von 2:1, 3:1 oder 4:1 sind.

11. *In-vitro*-Diagnosekit für das Rückfallrisiko des Krebses eines an der Atmung beteiligten Organs und/oder der Aggressivität des Krebses eines an der Atmung beteiligten Organs, um einen prognostischen Wert für die therapeutische Anpassung eines Krebses eines an der Atmung beteiligten Organs zu definieren, wobei das an der Atmung beteiligte Organ aus Lunge, Kehlkopf, Rachen, Mund, Nase, Kehle, Zunge, Nebenhöhlen, Luftröhre und Speicheldrüsen einschließlich Mandeln und Ohrspeicheldrüse ausgewählt ist,
wobei das Kit mindestens zwei Lektine, ausgewählt aus der Mischung aus GSL-I und GSL-II oder der Mischung aus UEA-1 und GSL-I, wobei die Lektine mit Biotin konjugiert sind, und mit Streptavidin funktionalisierte Magnetkugeln und gegebenenfalls mindestens zwei Lektine, ausgewählt aus UEA-1 oder seinem Homologen TJA-II, ABA, ACA, Jakalin, GSL-I und GSL-II, insbesondere das Gemisch aus GSL-I und GSL-II oder das Gemisch aus UEA-1 und GSL-I, konjugiert mit einem Fluorophor, einem Radioisotop, einem Enzym oder Goldkugeln, umfasst oder
wobei das Kit mindestens zwei Lektine, ausgewählt aus der Mischung aus GSL-I und GSL-II oder der Mischung aus UEA-1 und GSL-I, wobei die Lektine mit einem Fluorophor konjugiert sind, und gegebenenfalls mindestens zwei Lektine, ausgewählt aus UEA-1 oder seinem Homologen TJA-II, ABA, ACA, Jakalin, GSL-I und GSL-II, insbesondere das Gemisch aus GSL-I und GSL-II oder das Gemisch aus UEA-1 und GSL-I, konjugiert mit Biotin, einem Radioisotop, einem Enzym oder Goldkugeln, umfasst.

12. Diagnosekit nach Anspruch 11, wobei die mindestens zwei Lektine in gleicher oder ungleicher Menge sind.

13. Diagnosekit nach einem der Ansprüche 11 bis 12, wobei die mindestens zwei Lektine in nicht äquimolarer Menge sind,
wobei die 2 Lektine vorzugsweise UEA-1 und GSL-I in nicht äquimolarer Menge in einem Gewichtsverhältnis von 2:1, 3:1 oder 4:1 sind.

## Claims

1. An *in vitro* method for isolating cancer stem cells of organs involved in respiration in a biological sample, comprising:
(a) a step of labelling cancer stem cells of organs involved in respiration with at least one lectin selected from the lectins UEA-1 or its homologue TJA-II, ABA, ACA, jacalin, GSL-I, and GSL-II, said lectin being conjugated to biotin or a fluorophore, to obtain a biological sample in which cancer stem cells of organs involved in respiration are labelled by at least one lectin,
followed by
(b) a step of isolating said cancer stem cells of organs involved in respiration labelled by at least one lectin,
said isolation step being performed via a support functionalized with streptavidin or avidin constituted by magnetic beads and in the presence of a magnet, when said lectin is conjugated to biotin,
and said isolation step being carried out by cell sorting in flow cytometry, when said lectin is conjugated to a fluorophore,
wherein said organs involved in respiration are selected from the lungs, larynx, pharynx, mouth, nose, throat, tongue, sinuses, trachea, and salivary glands including the tonsils and parotid gland.

2. The *in vitro* isolation method according to claim 1, wherein said isolation step is followed by a detection step, said detection step comprising:
(a) a step of labelling cancer stem cells of organs involved in respiration with at least one lectin selected from UEA-1 lectin or its homologue TJA-II, ABA, ACA, jacalin, GSL-I, and GSL-II, said lectin being conjugated to a marker selected from: a fluorophore, a radioisotope, an enzyme, gold beads, or biotin, to obtain a biological sample in which cancer stem cells of organs involved in respiration are labelled by at least one lectin,
followed by
(b) a step of detecting said cancer stem cells of organs involved in respiration labelled by at least one lectin,
wherein said organs involved in respiration are selected from the lungs, larynx, pharynx, mouth, nose, throat, tongue, sinuses, trachea, and salivary glands including the tonsils and parotid gland.

3. The *in vitro* isolation method according to any of claims 1 to 2, wherein said at least one lectin is
at least two lectins selected from UEA-1 or its homologue TJA-II, ABA, ACA, jacalin, GSL-I, and GSL-II, in particular the mixture of GSL-I and GSL-II or the mixture of UEA-1 and GSL-I.

4. The *in vitro* isolation method according to any one of claims 1 to 3, wherein at least two lectins are used, said at least two lectins being in equal or unequal amounts.

5. The *in vitro* isolation method according to one of claims 1 to 3, in which at least two lectins are used, said at least two lectins being in non-equimolar quantities,
said two lectins preferably being UEA-1 and GSL-I in non-equimolar amounts in a weight ratio of 2:1, 3:1, or 4:1.

6. Use of at least one lectin selected from the lectins UEA-1 or its homologue TJA-II, ABA, ACA, jacalin, GSL-I, and GSL-II in an *in vitro* diagnostic method for the risk of recurrence of cancer of an organ involved in respiration and/or the aggressiveness of cancer of an organ involved in respiration to define a prognostic value for the therapeutic adaptation of cancer of an organ involved in respiration,
wherein said organ involved in respiration is selected from the lungs, larynx, pharynx, mouth, nose, throat, tongue, sinuses, the trachea, and the salivary glands including the tonsils and the parotid gland, wherein the diagnostic method comprises a step of labelling the cancer stem cells of organs involved in respiration of a biological sample of organs involved in respiration with at least one lectin selected from UEA-1 lectin or its homologue TJA-II, ABA, ACA, jacalin, GSL-I, and GSL-II, to obtain cancer stem cells of organs involved in respiration labelled with at least one lectin in said sample.

7. Use according to claim 6, wherein the diagnostic method comprises the steps of:
(a) labelling said cancer stem cells of organs involved in respiration with at least one lectin selected from UEA-1 lectin or its homologue TJA-II, ABA, ACA, jacalin, GSL-I, and GSL-II, to obtain cancer stem cells of organs involved in respiration labelled with at least one lectin in said biological sample,
said lectin being conjugated to a label selected from a fluorophore or biotin,
(b) Isolation of cancer stem cells of organs involved in respiration labelled by said at least one conjugated lectin:
- when labelling with a biotin-conjugated lectin, said isolation is performed via a support functionalized with streptavidin or avidin, in particular said functionalized support is constituted by magnetic beads functionalized with streptavidin or avidin and said isolation is performed by magnetic cell sorting in the presence of a magnet, or
- when labelling with a lectin conjugated to a fluorophore, said isolation is performed by cell sorting in flow cytometry,
(c) New labelling of cancer stem cells of organs involved in respiration isolated with at least one recognizing lectin chosen from among the lectins UEA-1 or its homologue TJA-II, ABA, ACA, jacalin, GSL-I, and GSL-II, to obtain isolated and labelled cancer stem cells of organs involved in respiration,
said lectin being conjugated to a label selected from a fluorophore, a radioisotope, an enzyme, gold beads, or biotin,
(d) Detection of said isolated and labelled cancer stem cells of organs involved in respiration with
- fluorescence microscopy or a fluorescence reader when the lectin is conjugated to a fluorophore, or when the lectin is conjugated to biotin and is detected via a fluorophore conjugated to streptavidin or avidin;
- luminescence microscopy or luminescence reader when the lectin is conjugated to an enzyme using a chemiluminescent substrate, or when the lectin is conjugated to biotin and is detected via an enzyme using a chemiluminescent substrate conjugated to streptavidin or avidin
- gamma camera when the lectin is conjugated to a radioisotope, or when the lectin is conjugated to biotin and is detected via a radioisotope conjugated to streptavidin or avidin;
- UV/visible microscopy or absorbance reader when the lectin is conjugated to an enzyme using a chromogenic substrate, or when the lectin is conjugated to biotin and is detected via an enzyme using a chromogenic substrate conjugated to streptavidin or avidin;
- Electron microscopy when the lectin is conjugated to gold beads, or when the lectin is conjugated to biotin and is detected via gold beads conjugated to streptavidin or avidin;
(e) Possibly quantification of cancer stem cells of organs involved in respiration;
(f) Comparison of the intensity of detection of cancer stem cells of organs involved in respiration in said biological sample with the intensity of detection of cancer stem cells of organs involved in respiration in a healthy sample adjacent to the biological sample,
and optionally comparison of the quantification of cancer stem cells of organs involved in respiration in said biological sample with the quantification of cancer stem cells of organs involved in respiration in a healthy sample adjacent to the biological sample,
(g) Deduction of the risk of recurrence of cancer of an organ involved in respiration and/or the aggressiveness of cancer of an organ involved in respiration to define a prognostic value for the therapeutic adaptation of cancer of an organ involved in respiration from the presence and possibly the quantity of cancer stem cells of organs involved in respiration.

8. Use according to claim 6, wherein the diagnostic method according to claim 6 comprises the steps of:
(a) labelling said cancer stem cells of organs involved in respiration with at least one lectin selected from UEA-1 or its homolog TJA-II, ABA, ACA, jacalin, GSL-I, and GSL-II lectins, to obtain cancer stem cells of organs involved in respiration labelled with at least one lectin in said biological sample,
said lectin being conjugated to a label selected from a fluorophore, a radioisotope, an enzyme, gold beads, or biotin,
(b) Detection of said cancer stem cells of organs involved in respiration labelled by
- fluorescence microscopy or fluorescence reader when the lectin is conjugated to a fluorophore or when the lectin is conjugated to biotin and is detected via a fluorophore conjugated to streptavidin or avidin;
- luminescence microscopy or luminescence reader when the lectin is conjugated to an enzyme using a chemiluminescent substrate, or when the lectin is conjugated to biotin and is detected via an enzyme using a chemiluminescent substrate conjugated to streptavidin or avidin,
- gamma camera when the lectin is conjugated to a radioisotope, or when the lectin is conjugated to biotin and is detected via a radioisotope conjugated to streptavidin or avidin;
- UV/visible microscopy or absorbance reader when the lectin is conjugated to an enzyme using a chromogenic substrate, or when the lectin is conjugated to biotin and is detected via an enzyme using a chromogenic substrate conjugated to streptavidin or avidin;
- Electron microscopy when the lectin is conjugated to gold beads, or when the lectin is conjugated to biotin and is detected via gold beads conjugated to streptavidin or avidin;
(c) Possibly quantification of cancer stem cells of organs involved in respiration;
(d) Comparison of the intensity of detection of cancer stem cells of organs involved in respiration in said biological sample with the intensity of detection of cancer stem cells of organs involved in respiration in a healthy sample adjacent to the biological sample,
and possibly comparing the quantification of cancer stem cells of organs involved in respiration in said biological sample with the quantification of cancer stem cells of organs involved in respiration in a healthy sample adjacent to the biological sample;
(e) Deduction of the risk of recurrence of cancer of an organ involved in respiration and/or the aggressiveness of cancer of an organ involved in respiration to define a prognostic value for the therapeutic adaptation of cancer of an organ involved in respiration based on the presence and possibly the quantity of cancer stem cells of organs involved in respiration.

9. Use according to any one of claims 6 to 8, wherein said at least one lectin is
at least two lectins selected from UEA-1 or its homologue TJA-II, ABA, ACA, jacalin, GSL-I, and GSL-II, in particular the mixture of GSL-I and GSL-II or the mixture of UEA-1 and GSL-I.

10. Use according to any of claims 6 to 9, wherein at least two lectins are used, said at least two lectins being in equal or unequal or non-equimolar amounts,
said two lectins preferably being UEA-1 and GSL-I in non-equimolar amounts in a weight ratio of 2:1, 3:1, or 4:1.

11. *In vitro* diagnostic kit for the risk of recurrence of cancer of an organ involved in respiration and/or the aggressiveness of cancer of an organ involved in respiration to define a prognostic value for the therapeutic adaptation of cancer of an organ involved in respiration, wherein said organ involved in respiration is selected from the lungs, larynx, pharynx, mouth, nose, throat, tongue, sinuses, trachea, and salivary glands including the tonsils and parotid gland,
the kit comprising at least two lectins selected from the mixture of GSL-I and GSL-II or the mixture of UEA-1 and GSL-I, said lectins being conjugated to biotin, and magnetic beads functionalized with streptavidin, and optionally at least one of the following: at least two lectins selected from UEA-1 or its homologue TJA-II, ABA, ACA, jacalin, GSL-I, and GSL-II, in particular the mixture of GSL-I and GSL-II or the mixture of UEA-1 and GSL-I, conjugated to a fluorophore, a radioisotope, an enzyme, or gold beads, or
the kit comprising at least two lectins selected from the mixture of GSL-I and GSL-II or the mixture of UEA-1 and GSL-I, said lectins being conjugated to a fluorophore, and optionally at least two lectins selected from UEA-1 or its homologue TJA-II, ABA, ACA, jacalin, GSL-I and GSL-II, in particular the mixture of GSL-I and GSL-II or the mixture of UEA-1 and GSL-I, conjugated to biotin, a radioisotope, an enzyme or gold beads.

12. Diagnostic kit according to claim 11, said at least two lectins being in equal or unequal quantities.

13. Diagnostic kit according to one of claims 11 to 12, said at least two lectins being in non-equimolar quantities,
said two lectins preferably being UEA-1 and GSL-I in non-equimolar quantities in a weight ratio of 2:1, 3:1, or 4:1.
